# EUROPÄISCHE PATENTSCHRIFT

(11) **EP 2 844 234 B1**
(45) Veröffentlichungstag und Bekanntmachung des Hinweises auf die Patenterteilung: **08.01.2020**
(21) Anmeldenummer: 13713755.0
(22) Anmeldetag: 02.04.2013
(51) Int. Cl.: A61K 9/28

(54) **TABLETTEN MIT ÜBERZUG UND DEREN HERSTELLUNG**
TABLETS WITH COATING AND THE PRODUCTION THEREOF
COMPRIMÉS POURVUS D'UN ENROBAGE ET LEUR FABRICATION

(30) Priorität: 27.04.2012 EP 12002988; 11.09.2012 EP 12006370
(43) Veröffentlichungstag der Anmeldung: 11.03.2015
(73) Patentinhaber: Merck Patent GmbH, 64293 Darmstadt (DE)
(72) Erfinder: OGNIBENE, Roberto, 64297 Darmstadt (DE); BERNHARDT, Sandra, Erika, 64367 Muehltal (DE); BREIDUNG, Melanie, Mechthild, 64832 Babenhausen/Hergershausen (DE); LUBDA, Dieter, 64625 Bensheim (DE); OHREM, Hans-Leonhard, 64342 Jugenheim (DE)
(86) Internationale Anmeldenummer: PCT/EP2013/000971
(87) Internationale Veröffentlichungsnummer: WO 2013/159861

(56) Entgegenhaltungen:
- EP-A1- 2 415 466
- WO-A1-2009/152922
- WO-A2-2009/135646

## Beschreibung

Durch die vorliegende Erfindung wird eine schnell zerfallende pharmazeutische Formulierung in Form einer mit einem Überzug versehenen Tablette mit erhöhter mechanischer Festigkeit, bzw. Härte zur Verfügung gestellt. Weiterhin ist ein Verfahren zur Herstellung der überzogenen Tablette Gegenstand der Erfindung sowie die Verwendung dieser Formulierungen.

Mit einem Überzug versehene Tabletten sind seit langem bekannt. Der Überzug dient u. a. zum Schutz vor nachteiligen physikalischen und mechanischen Einflüssen der in den Formulierungen enthaltenen Wirkstoffbestandteile. Solche negativen äußere Einflüsse können beispielsweise hervorgerufen werden durch UV-Licht, Sauerstoff, oder Feuchtigkeit, aber auch durch mechanische Belastung, wie Stoß und Reibung, wodurch sich Wirk- und Hilfsstoffe zersetzen können oder durch Abrieb der Tabletten die Dosierung nicht mehr gewährleistet ist.

Prinzipiell wird eine Lackierung von Tabletten aus den folgenden Gründen vorgenommen:
- Identifizierung und Wiedererkennungswert auf dem Markt und bei den Kunden durch spezielle Farben,
- Geschmacksmaskierung für unangenehm schmeckende Arzneistoffe,
- Überzug für eine verzögerte und/oder gezielte Freisetzung (z.B. magensaftresistent),
- Verbesserung der Oberflächenbeschaffenheit, durch die sich Vorteile bei der Verpackung ergeben und die Tabletten sich vom Verwender besser schlucken lassen.

Erfahrungsgemäß können Tabletten durch einen geeigneten Schutz-Überzug vor äußeren Einflüssen bewahrt werden. Entsprechende Überzüge können aus einer Glasur bestehen, die zur Geschmacksverbesserung Zucker enthalten und gegebenenfalls eingefärbt sein kann. Letzteres kann aus ästhetischen Gründen erfolgen, um die als Tabletten vorliegende Formulierung markttechnisch hervorzuheben.

Außer zuckerhaltigen Überzügen sind auch Glasuren bekannt, die aus einem für den Menschen unbedenklichen natürlichen oder synthetischen Polymerfilm bestehen. Letztere können beispielsweise aus Gelatine, Methylcellulose, Polyvinylpyrrolidon, Polyvidonacetat oder anderen verträglichen Polymeren bestehen, die je nach dem vorgesehenen Wirkungsort des Arzneimittels schnell löslich oder verzögert sich erst im Magen oder Dünndarm lösen. Zur Verbesserung der Löslichkeit des Überzugs können die Polymere in Kombination mit einer geringen Menge einer löslichen Komponente eingesetzt werden, so dass einerseits die filmbildenden Eigenschaften des Polymers genutzt werden können und andererseits sichergestellt ist, dass der Film bzw. Überzug sich wenigstens teilweise in der Mundhöhle in Gegenwart von Feuchtigkeit löst und so der Tablettenkern für Feuchtigkeit zugänglich wird und wie gewünscht schnell zerfällt. Im Handel sind verschiedene fertige Lösungen zum Auftrag von Glasuren bzw. Filmen auf Tabletten erhältlich.

Als sogenannte Filmtabletten werden häufig Tabletten für die orale Applikation von Medikamenten, Vitaminen und insbesondere für schnell wirkende Arzneimitteln angeboten. Die durch den Überzug sich bildende glatte Oberfläche kann zusammen mit der Wahl einer günstigen Form die Einnahme der Tabletten erleichtern, sodass sie sich besser schlucken lassen. Gleichzeitig kann der Überzug, wie oben schon erwähnt, einer Geschmacksmaskierung für unangenehm schmeckende Arzneimittel dienen. Wie bereits angedeutet, trägt er auch zur Erhöhung der Stoßfestigkeit und der Wasserresistenz des gepressten Tablettenkerns bei. Die verbesserte Stoßfestigkeit ist vorteilhaft während der maschinellen Verpackung der Tabletten, insbesondere wenn die Tabletten in Folien, sogenannte Blister verpackt werden. Durch den Überzug wird ein Abrieb vermieden. Manche besonders weiche Tablettenkerne lassen sich erst nach dem Auftrag eines geeigneten Überzugs verpacken.

Darüber hinaus kann eine Färbung oder Markierung des Tablettenüberzugs dazu beitragen, die Wiedererkennung zu erhöhen und Verwechslungen von Tabletten vorzubeugen und zu vermeiden, was beispielsweise für ältere oder demenzkranke Patienten zur Erkennung der Tabletten mit bestimmtem pharmazeutischen Wirkstoffen wesentlich ist. Sie kann aber auch durch den Einsatz von speziellen Farben der Arzneimittelsicherheit dienen. Solche in dem Überzug enthaltene Farben können in der Tablette enthaltene lichtempfindliche Wirk- und Hilfsstoffe schützen. Dementsprechend bieten entsprechende Tablettenüberzüge für die im Tablettenkern enthaltenen Substanzen Schutz vor äußeren Einflüssen, wie Licht, Wärme, Feuchtigkeit, aber auch für die Tablette selbst gegen mechanischen Abrieb.

Damit Tabletten überhaupt in einem solchen Beschichtungsverfahren behandelt werden können, müssen die Tabletten eine entsprechende Festigkeit aufweisen und dürfen nur einen sehr geringen Abrieb zeigen.

In den letzten Jahren sind dementsprechend für vielfache Applikationsgebiete sogenannte ODTs ("oral dispersible tablets") immer beliebter geworden, die einerseits bei Feuchtigkeitskontakt (z.B. Speichel), aufgrund enthaltener Zusatzstoffe, wie z.B. Sprengmittel (Superdisintegrants), Polymere, Stärken, usw., schnell im Mund zerfallen, andererseits aber auch die Voraussetzungen erfüllen müssen, wenn die hergestellten Tablettenkerne mit einem Überzug versehen werden sollen.

Im Handel werden für diesen Zweck unterschiedliche Mischungen, sogenannte Ready-to-use-Hilfsstoffsysteme, für die Herstellung von oral schnell zerfallenden Tabletten angeboten. In der Regel sind damit nur Tabletten mit niedrigen Härten herstellbar, wobei die niedrigen Härten von einem relativ hohen Abrieb begleitet sind. Entsprechende ODTs sind feuchtigkeitsempfindlich, so dass sie den in der Pharmazeutischen Industrie üblichen wässrigen Beschichtungsmethoden nicht zugänglich sind. Folglich ist mit diesen Mischungen die Herstellung von beschichteten Tabletten nicht möglich.

Außerdem kann in den Beschichtungsanlagen durch die geringe Festigkeit ein nicht akzeptabler Abrieb erzeugt werden, der evtl. mit einer starken Staubbildung verbunden ist. Weiterhin bewirkt ein hoher Abrieb, dass die Tabletten nach der Beschichtung an der Oberfläche keinen vollständigen funktionalen bzw. ästhetischen Überzug aufweisen. Auf der Oberfläche so hergestellter Tabletten können die abgeriebenen Partikel deutlich sichtbar sein, die durch den Lack an der Oberfläche fixiert und dort wieder verklebt worden sind. Weiterhin können entsprechende Tabletten stark beschädigt werden durch Abplatzen oder Abbrechen von relativ großen Stücken aus der Oberfläche, wodurch vorhandene Facetten, Kanten, Schriftzüge eventuell stark beschädigt werden können.

Andererseits weisen überzogene Tabletten nicht nur Vorteile auf. Sollen sich die verabreichten Tabletten direkt nach der Einnahme im Mund schnell lösen, kann hier der Überzug hinderlich sein. Durch den Überzug können sich die Zerfallszeiten deutlich verlängern, da die Lackierung der Tablettenkerne üblicherweise mit einer Nachhärtung verbunden ist. Außerdem können die im Tablettenkern enthaltenen Wirkstoffe während der Lackierung und der Lagerung mit den Inhaltsstoffen des Überzugs in Wechselwirkung treten und gegebenenfalls unter Bildung von unerwünschten Nebenprodukten reagieren.

Daher ist die Zusammensetzung der Ausgangslösung zur Herstellung des Überzugs in geeigneter Weise zu wählen, sodass sich der gebildete Überzug zumindest teilweise lösen oder Risse bilden kann, damit sich durch die Feuchtigkeit des Speichels der Tablettenkern lösen oder schnell zerfallen kann. Trotzdem muss der Überzug lagerstabil sein, sodass entsprechende Tabletten sich bei Lagerung bei erhöhter Luftfeuchtigkeit nicht zersetzen oder bei Kontakt mit geringen Mengen an Feuchtigkeit nicht zerfallen. Letzteres würde die Dosierung der Tabletten nicht mehr gewährleisten und eine Einnahme durch den Patienten wäre dadurch ebenfalls nicht mehr möglich.

Um einerseits ein schnelles Zerfallen im Mund trotz des Überzugs der Tablette zu gewährleisten, andererseits aber auch die Vorteile des Überzugs für die Stabilität der Tabletten nutzen zu können, sind durch die Entwickler verschiedene Lösungen vorgeschlagen worden.
In EP 2 433 621 A1 werden beispielsweise Überzüge mit Aussparungen in den Überzügen vorgeschlagen, die sich in Rillen befinden und während der Herstellung des Überzugs erzeugt werden. Nachteilig ist jedoch, dass für die Herstellung des Überzugs ein spezielles Verfahren notwendig ist und letzteres nicht ohne weiteres in vorhandenen Apparaturen durchführbar ist.

Der Abrieb der Tabletten ist jedoch nicht nur während der Lagerung von Bedeutung. Insbesondere spielt er während der Herstellung des gewünschten Überzugs eine Rolle, da durch einen starken Abrieb der Tabletten davon auszugehen, dass der Wirkstoffgehalt und die gewünschte Einzeldosis der verabreichten Tablette nicht mehr gewährleistet werden kann. Dieses kann bei einer Unterdosierung des Wirkstoffes durch zu hohen Verlust durch Abrieb zu gravierenden Folgen führen, weil eine optimale Dosierung des pharmazeutischen Wirkstoffs nicht erreicht wird. Gegebenenfalls könnte es auch zu einer Überdosierung des Wirkstoffs führen, wenn Abrieb im Überzug von nicht beschädigten Tabletten wieder eingebunden wird. Beides sollte unbedingt vermieden werden. Auch eine vorzeitige Zersetzung bzw. ein Zerfall von OD-Tabletten während der Lagerung durch Feuchtigkeit oder während der Herstellung des Überzugs würde die Eigenschaften der Tabletten verändern und nicht reproduzierbare Herstellmethoden zur Folge haben.

In der Patentanmeldung WO 2009/135646A2 wird versucht, schnell zerfallende, wirkstoffhaltige Tabletten herzustellen, indem Partikel eines geeigneten Trägermaterials mit einer tensidfreien, wirkstoffhaltigen Coatinglösung besprüht und granuliert wird. Dieses erhaltene Granulat kann anschließend durch Verpressen tablettiert werden. Die erhaltenen Tabletten werden gegebenenfalls noch mit einem geeigneten Überzug versehen werden. Durch das Granulieren der Trägerpartikel mit einer wirkstoffhaltigen Coatinglösung wird versucht, den Zusammenhalt der Granulatpartikel in den Tabletten zu verbessern und damit die Friabilität der Tabletten zu verringern. Nach dem Verpressen ist der Wirkstoff homogen in der Tablette verteilt, jedoch ist ein Nachteil dieses Vorgehens, dass durch die Granulierung durch Aufsprühen zusätzlich außer dem Wirkstoff in einer Lösung mindestens eine Coatingkomponente und weitere Hilfsmittel, wie eine Base und ein Bindemittel, auf die Partikel des Trägermaterials aufgesprüht werden, wodurch die Zahl der Inhaltsstoffe der Tablette erhöht wird. Letzteres ist jedoch nicht immer erwünscht, weil sich dadurch Wechselwirkungen mit dem Wirkstoff ergeben können. Auch wird durch den Überzug die Zerfallszeit der Tabletten verlängert.

Vergleichbar aufwändig wird in EP 2 415 466 A1 vorgegangen. In diesem Fall wird Mannitpulver mit Crospovidon, einem Zerfallsmittel, granuliert. Das erhaltene Granulat wird dann mit einem Granulat aus einem körnigem Mannit, wie z. B. wie Perlitol® 300DC, und Nalfurafin als Wirkstoff, vermischt, und die resultierende Mischung wird tablettiert. Zur Herstellung der Mischung wird granuliertes Mannitpulver mit körnigem Mannit im Verhältnis von etwa 1: 9 bis 1: 1 bezogen auf das Gewicht verwendet. Zur Herstellung dieser Mischung werden darüber hinaus spezielle Mannitqualitäten eingesetzt, und zwar Perlitol® 50C mit einer mittleren Partikelgröße von 50 µm und Perlitol® 300DC mit einer mittleren Partikelgröße von 250 µm. Die erhaltenen Tablettenkerne können ebenfalls mit einem Überzug versehen werden.

### Aufgabenstellung

Aufgabe der vorliegenden Erfindung ist es daher, eine Zusammensetzung zur Herstellung von Tabletten zur Verfügung zu stellen, Tablettenkerne mit hoher Härte und geringem Abrieb herstellbar sind, die gleichzeitig in Gegenwart von Feuchtigkeit, insbesondere im Mund, schnell zerfallen und die sich problemlos nahezu ohne Veränderung mit einem stabilisierendem Überzug versehen lassen, durch den sich jedoch die Zerfallszeit der Tablette mit Überzug im Vergleich zu der des Tablettenkerns möglichst wenig verändert. Der schnell zerfallende Kern einer solchen Tablette sollte aus diesem Grund ebenso wie bekannte Produkte einfach herstellbar sein und möglichst aus den Trockenmischungen der Ausgangsstoffe direkt tablettierbar sein. Die hergestellten Tabletten sollten eine hohe mechanische Festigkeit besitzen, so dass sie in der nachfolgenden Behandlung, wie z. B. während der Herstellung des Überzugs und während der Verpackung, dem Transport und gegebenenfalls beim Herausdrücken aus der Verpackung unbeschädigt bleiben.

Insbesondere ist es daher Aufgabe der vorliegenden Erfindung, eine Kombination aus hartem Tablettenkern mit geringem Abrieb und einem geeigneten Überzug zur Verfügung zu stellen, wodurch gleichmäßig überzogene, wirkstoffhaltige Tabletten herstellbar sind, welche hohe Härten aufweisen und bei Verabreichung im Mund des Patienten schnell zerfallen. Weiterhin ist es Aufgabe der Erfindung, ein geeignetes, einfach durchführbares Verfahren zur Verfügung zu stellen, durch das auf den schnell zerfallenden Tablettenkernen bei möglichst geringem Abrieb unter Erhalt der Tabletteneigenschaften überzogene Tabletten erhalten werden, die bei Einnahme im Mund weiterhin schnell zerfallen.

### Lösung der Aufgabe

Durch die vorliegende Erfindung können überraschender Weise pharmazeutische Formulierungen in Form einer Tablette mit Überzug zur Verfügung gestellt, die in Gegenwart von Feuchtigkeit schnell zerfällt und die aus einem Tablettenkern besteht,
a) der erhältlich ist aus einer homogenisierten, direkt verpressbaren Co-Mischung von sprühgranuliertem Mannit und quervernetzter Croscarmellose-Natrium und mindestens einem pharmazeutischen Wirkstoff oder Nahrungsergänzungsmittel und Additiven,
   und
b) einem Überzug, der in Form einer wässrigen oder einer Wasser und Alkohol haltigen Lösung aufgebracht und getrocknet wird.

Zur Herstellung des Tablettenkerns dieser pharmazeutischen Formulierung wird eine Co-Mischung, bestehend aus 90 bis 95 Gew.-% Mannit und 3 bis 7 Gew.-% Croscarmellose-Natrium als Tablettensprengmittel und gegebenenfalls bis zu 1 Gew.-% Magnesiumstearat, verwendet.

Entsprechende Tabletten sind ausgezeichnet als sogenannte IR- oder FR-Tabletten geeignet.

Die zur Herstellung des Tablettenkerns verwendete Co-Mischung weist einen Fließwinkel im Bereich von 33 bis 38°, Partikelgrößen im Bereich von 70 bis 120 µm (Dᵥ₅₀; Laser), eine Schüttdichte im Bereich von 0,55 bis 0,65 g/ml und eine Stampfdichte im Bereich von 0,70 bis 0,80 g/ml auf. Als besonders vorteilhaft wirkt sich die große BET-Oberfläche der Co-Mischung im Bereich von 2,4 bis 3,5 m²/g aus. Der verwendete Tablettenkern kann erfindungsgemäß einen pharmazeutischen Wirkstoff oder Nahrungsergänzungsmittel in einer Menge von 0,1 bis zu 50 Gew-% bezogen das Gewicht des Tablettenkerns enthalten. Zur Herstellung der erfindungsgemäßen schnell zerfallenden Tablette wird der Überzug in Form einer Wasser bzw. Wasser-Ethanol haltigen Lösung aufgebracht, worin zur bildung des Überzugs lösliche Filmbildner aus der Gruppe Polyvinylpyrrolidon, Vinylpyrrolidon / Vinylacetat Copolymer, Polyvinylacetat, Hydroxypropylmethylcellulose, Methacrylestercopolymer oder deren Mischungen enthalten sind. In der Überzugslöung (CoatingLösung) können weitere Substanzen enthalten sein, die sich bereits während des Lösens des äußeren Tablettenüberzugs im Mund angenehmen auswirken. Hierbei kann es sich um einen oder mehrere Zucker aus der Gruppe Glucose, Dextrose, Fructose, Laktose, Maltose, Xylose, Sucrose, Maissirup, Sorbit, Hexitol, Maltit, Xylit und Mannit, gegebenenfalls mindestens einen Polyalkohol ausgewählt aus der Gruppe Glycerin, Polyethylenglykol und Propylenglykol, sowie gegebenenfalls mindestens eine essbare, für Lebensmittel geeignete Säure aus der Gruppe Zitronensäure, Äpfelsäure, Weinsäure, Fumarsäure, Phosphorsäure, Oxalsäure und Ascorbinsäure, und Aromaöle und/oder Geschmacksstoffe und gegebenenfalls eines Süßstoffs, wie z. B. Sucralose, Aspartam, Acesulfam-K oder ähnliche handeln.

Erfindungsgemäß können die in Gegenwart von Feuchtigkeit schnell zerfallenden überzogenen Tabletten mindestens einen Wirkstoff ausgewählt aus der Gruppe Atypical antipsychotics, Antipsychotics, Antidepressants, Anti-histamines, Acetylcholinesterase inhibitors, Analgesics, Anti-pyretics, Anticonvulsant, Anticholinergic, Antiemetics, Benzodiazepines, Corticosteroids, DDC inhibitors [carbidopa], Dopamine receptor antagonists, Monoamine oxidase inhibitors (MAOIs), Nonbenzodiazepine Hypnotics, Opioid analgesic [Tramadol], Proton pump inhibitors, Triptans/Serotonin agonists, NSAIDs und SSRIs enthalten.

Für die so hergestellten pharmazeutischen Formulierungen hat es sich als besonders vorteilhaft herausgestellt, dass der Tablettenkern ohne Überzug einen niedrigen Abrieb von weniger als 0,50 % bezogen auf das Gewicht aufweist, da es hierdurch möglich ist, die gewünschten Überzüge sehr gleichmäßig aufzutragen und die hergestellten Tabletten eine sehr gleichmäßige Oberfläche aufweisen.

Während es bisher als unmöglich galt, in Gegenwart von Feuchtigkeit schnell zerfallende Tabletten durch wasserhaltige Lösungen mit einem Überzug zu versehen ist es durch das erfindungsgemäße Verfahren unter Verwendung der beschriebenen Tablettenkerne möglich, entsprechende pharmazeutische Formulierungen herzustellen, indem die vorab unter Verwendung der oben genannten Co-Mischung hergestellten Tablettenkerne in einer Lackiertrommel unter Durchmischung auf eine erhöhte Temperatur erwärmt werden und der Überzug durch Aufsprühen der niedrigviskosen Überzugslösung und Trocknen bei erhöhter Temperatur auf den Tablettenkernen erzeugt wird. Hierbei werden die Tablettenkerne vor dem Aufsprühen der Überzugslösung auf eine Temperatur im Bereich von 35 bei 60 °C erwärmt werden. Besonders gute Ergebnisse werden erzielt, wenn auf eine Temperatur im Bereich von 40 bis 55°C erwärmt wird und die Tabletten nach dem Aufsprühen der Überzugslösung 10 bis 20 Minuten nachgetrocknet werden.

### Detaillierte Beschreibung der Erfindung

Wie bereits beschrieben, werden Tabletten, die schnell im Mund schnell zerfallen, für die orale Applikation immer beliebter. Solche Tabletten sollten im Mund innerhalb von weniger als 90, vorzugsweise weniger als 60 Sekunden, besonders bevorzugt in einer Zeit von nicht mehr als 30 Sekunden, in der Mundhöhle zerfallen, wobei sie ein angenehmes Mundgefühl erzeugen und gut schmecken sollten. Nach neuesten Anforderungen sollte eine solche schnell zerfallende Tablette eine Zerfallszeit von weniger als 18 Sekunden aufweisen. Letzteres stellt in den meisten Fällen den Entwickler (Fachmann) vor große und zum Teil vor nicht lösbare Herausforderungen.

Der schnell zerfallende Kern einer solchen Tablette sollte ebenso wie bekannte Produkte einfach herstellbar sein und möglichst aus den Trockenmischungen der Ausgangsstoffe direkt tablettierbar sein. Die hergestellten Tabletten sollten eine hohe mechanische Festigkeit besitzen, so dass sie in der nachfolgenden Behandlung während der Verpackung, dem Transport und gegebenenfalls beim Herausdrücken aus der Verpackung unbeschädigt bleiben.

Allgemein ist es daher erstrebenswert, dass während des Beschichtungsprozesses der Abrieb des Tablettenkerns kleiner 0,2 bis 0,4 Gew.-% ist.

An sich sind für die Herstellung schnell zerfallender Tabletten im Handel verschiedenste Fertigmischungen erhältlich, aus denen sich gemeinsam mit dem gewünschten Wirkstoff schnell zerfallende Tabletten herstellen lassen. Es können darin verschiedenste, in der Pharmazeutischen Industrie verwendete Tablettierhilfsmittel enthalten sein, die jedoch immer mit einer Substanz kombiniert sind, welche mit der Feuchtigkeit im Mund reagiert und zum Quellen und Aufplatzen, d. h. zum Zerfallen der Tablette führen. Als Träger für die Herstellung der wirkstoffhaltigen Tabletten sind in entsprechenden Fertigmischungen bevorzugt hydroxylgruppenhaltige, natürliche Substanzen einsetzbar. Solche Trägersubstanzen, häufig auch als Excipients bezeichnet, sind Polyole, wie Mannit, Xylit, Sorbit, Erythrit, aber auch Laktose oder Stärke und ihre Derivate oder andere neutrale Stoffe, welche sich nicht auf die Aktivität des pharmazeutischen Wirkstoffs auswirken und bei dem Verpressen der Tabletten zu ausreichend harten Tabletten führen. Es ist jedoch nicht ohne weiteres möglich, die hergestellten, in Gegenwart von Feuchtigkeit schnell zerfallenden Tablettenkerne nach dem Verpressen mit Hilfe von wasserhaltigen Zusammensetzungen mit einem gewünschten Überzug versehen werden, da sich der Tablettenkern auch in Gegenwart von nur geringen Flüssigkeitsmengen verändern kann. Auch kann sich möglicher Abrieb nachteilig auf die Qualität des Überzugs auswirken kann.

Um aus den hergestellten, schnell zerfallenden Tablettenkernen Filmtabletten herzustellen, muß im allgemeinem mit folgenden Problemen, gegenüber einer "normalen" Tablette gerechnet werden:
- Deutlich längere Zerfallszeiten (verbunden mit der Nachhärtung nach der Lackierung) und dadurch eine nicht gewollte Verzögerung/Verlängerung der Freisetzung des Wirkstoffs
- Wechselwirkungen des Arzneistoffes mit der Lackschicht, damit verbunden die Entstehung von unerwünschten Nebenprodukten.

Durch Versuche ist bekannt, dass sich aus Fertigmischungen, wie sie in der Anmeldung WO 2009/152922 A1 beschrieben sind, bereits bei niedrigem Pressdruck, wie gewünscht, schnell zerfallende Tabletten mit hohen Härten herstellen lassen. Im Handel werden diese Fertigmischungen durch Merck (Darmstadt, Deutschland) unter dem Markennamen Parteck ODT® angeboten. Das Produkt weist einen Fließwinkel im Bereich von 33 bis 38° auf bei Partikelgrößen im Bereich von 70 bis 120 µm (D₅₀; Laser). Die Schüttdichte liegt im Bereich von 0,55 bis 0,65 g/ml bei einer Stampfdichte von 0,70 bis 0,80 g/ml. Gleichzeitig weist diese Fertigmischung eine hohe BET-Oberfläche im Bereich von 2,4 bis 3,5 m²/g auf. Dieses Produkt besteht aus 90 bis 95 Gew.-% Mannit und 3 bis 7 Gew.-% Croscarmellose-Natrium als Tablettensprengmittel.
Aus einer Mischung von Parteck ODT® mit bis zu 50 Gew.-% eines aktiven Wirkstoffs und gegebenenfalls 1 Gew.-% Magnesiumstearat oder Natriumstearylfumarate oder eines anderen üblicherweise verwendeten Schmier-oder Gleitmittels bezogen auf das Gesamtgewicht der Mischung lassen sich durch direktes Verpressen oral schnell zerfallende Tabletten mit niedrigen Friabilitäten und guten Eigenschaften während der Verpackung und Lagerung herstellen.

Andere Fertigmischungen werden beispielsweise unter den Namen Ludiflash®, Perlitol® Flash, Pharmaburst® 500 oder Prosolv® ODT vertrieben. Weitere Mischungen und ihre Verwendung sind in dem Review von B. G. Prajapati und N. Ratnakar, [Int. J. of. PharmTech Research, Vol. 1, No. 3, 790-798, (2009)] beschrieben. Von den genannten Fertigmischungen basieren insbesondere Parteck® ODT, Ludiflash®, und Perlitol® Flash auf Mannit als Hauptinhaltsstoff bzw. Trägermaterial, während Prosolv® ODT aus einer Mischung von mikrokristlliner Cellulose, kolloidalem Siliziumdioxid, jeweils 30 - 40 % Mannit und Fruktose, sowie Crospovidone als Tablettensprengmittel besteht.

Die von der Firma BASF (Deutschland) unter dem Markennamen Ludiflash®erhältliche Fertigmischung besteht aus D-Mannitol, Crospovidone, Polyvinylacetat, und geringen Mengen Povidone. Polyvinylacetat ist in die Formulierung eingebracht als Kollicat®SR 30 D, das aus einer Polyvinylacetat-Dispersion besteht, die mit Povidone stabilisiert ist. Bei dieser Zusammensetzung handelt es sich um ein weißes, freifließendes Pulver, das einen Fließwinkel von etwa 38° aufweist und dabei eine Partikelverteilung wie folgt aufweist:

| | |
|---|---|
| >0,400 mm | max. 20% |
| <0,200 mm | max. 90%, min. 45% |
| <0,063 mm | max. 45%, min. 15% |
| Schüttdichte: | 0,40 - 0,52 g/ml. |

Die Fertigmischung Perlitol® Flash der Firma Roquette besteht aus einer sprühgetrockneten Zusammensetzung aus 80 % Mannit und 20 % Maisstärke.

Die unter dem Markennamen Pharmaburst® von SPI Pharma vertriebenen Fertigmischungen wiederum 73 - 94% einer Polyol-Kombination, die aus nach Angaben der Firma aus Mannit und Sorbit besteht, sowie Tablettensprengmittel und Gleitmittel enthält.

F-Melt® Type C or M (Fuji Chemical Industry, Co., Ltd.) wiederum enthält etwa 65 Gew.-% Mannit, Xylit, microkristalline Cellulose, Crospovidone und andere Additive.

Von Pharmatrans SANAQ AG werden als weitere Fertigmischungen mit einem hohen Mannitgehalt OroCell® 200 & 400 angeboten. Die Mischungen haben einen Mannitgehalt von 90 % und unterscheiden sich dadurch, dass die erste Mischung einen mittleren Partikeldurchmesser von <315µm und die zweite einen mittleren Partikeldurchmesser von <500µm aufweist.

Die Fertigmischung der Firma Baker mit der Bezeichnung PanExcea® ODT MC200G besteht aus 75 % Mannit und 25 % Kalziumsilikat.

Durch Vergleichsversuche hat sich nun gezeigt, dass insbesondere entsprechende Fertigmischungen, die einen hohen Gehalt an sprühgetrocknetem, granuliertem Mannit als Trägermaterial enthalten, sich mit niedrigem Pressdruck zu Tabletten mit höheren Härten verpressen lassen, die im Test gleichzeitig vergleichsweise geringe Friabilitäten aufweisen. Wichtig scheint in diesem Zusammenhang die geeignete Kombination aus der Mannitqualität und dem in der Fertigmischung enthaltenen Tablettensprengmittel zu sein. Durch einen hohen Anteil an sprühgetrocknetem, granuliertem Mannit mit einer angepassten Partikelgrößenverteilung wird einerseits die Fließfähigkeit der Mischung vorwiegend durch die Eigenschaften des Mannits bestimmt, die wiederum wesentlich ist für einen problemlosen Tablettierungsprozess. Wichtig für die Tablettierung ist insbesondere auch die Kompressibilität aller in der Fertigmischung enthaltenen Komponenten, so dass mit möglichst niedrigem Pressdruck gearbeitet werden kann.
Unerwarteter Weise wurde gefunden, dass entsprechende mannithaltige Zusammensetzungen, insbesondere aber Zusammensetzungen, die gleichzeitig Croscarmellose-Natrium als Sprengmittel enthalten, wie z. B. die Fertigmischung Parteck ODT, sich bereits bei niedrigem Druck zu harten Tabletten verpressen lassen, wobei letztere gleichzeitig eine geringe Friabilität von weniger als 0,35 % zeigen, während für vergleichbare Zusammensetzungen nach derselben Verarbeitung teilweise lediglich eine Friabilität etwa 1 - 2% ermittelt werden kann.

Nach dem Verpressen können die hergestellten Tablettenkerne, die eine sehr große mechanische Stabilität und einen schnellen Zerfall aufweisen, überraschender Weise in einem nächsten Prozessschritt trotz des enthaltenen Tablettensprengmittels in einer geeigneten Beschichtungsanlage mit Überzug aus einer wasser- oder Wasser und Ethanol haltigen Zusammensetzung versehen werden, ohne die Zerfallseigenschaften der so behandelten Tablette wesentlich zu verändern, so dass der wirkstoffhaltige Tablettenkern anschließend vor äußeren Einflüssen geschützt ist. Diese Möglichkeit ist umso überraschender als bisher bekannte, schnell zerfallende Tabletten, in denen ein Überzug zum Schutz der enthaltenen Wirkstoffe erforderlich schien, nur aus überzogenen, wirkstoffhaltigen Partikeln hergestellt worden sind, wie z. B. in US 2011/0129530 A1 beschrieben. Und zwar werden darin die überzogenen, wirkstoffhaltigen Partikel mit weiterem Füllstoff und/oder Bindemittel, einem Tablettensprengmittel und mit einem Gleitmittel, wie Magnesiumstearat, vermischt und zu schnell zerfallenden Tabletten gepresst.

Die Vorstellung, dass oral schnell zerfallende Tabletten aufgrund ihrer Empfindlichkeit gegenüber Feuchtigkeit keinen äußeren Überzug aufweisen können sondern nur durch Verpressen der Ausgangsmischung herstellbar sind, hat sich auch in den Arzneibüchern niedergeschlagen. Beispielsweise werden in der Europäischen Pharmacopeia solche Tabletten als nicht überzogene Tabletten beschrieben, die im Mund schnell zerfallen, bevor sie geschluckt werden. (Ph. Eur., 2005).

Im Gegensatz dazu hat es sich insbesondere bei Verwendung, der in der Anmeldung WO 2009/152922 A1 beschriebenen Fertigmischungen, welche unter dem Markennamen Parteck® ODT vertriebenen werden, gezeigt, dass die Tablettenkerne nach dem Tablettieren überraschender Weise unter Verwendung von wasserhaltigen Zusammensetzungen mit einem Überzug versehen werden können und so überzogene Tabletten mit genügender oder auch erhöhter Festigkeit hergestellt werden können, die einen sehr niedrigen Abrieb aufweisen. Nach dem Aufbringen des Überzugs weisen die Tabletten weiterhin niedrige Zerfallszeiten auf. Unter bestimmten Bedingungen können die Zerfallszeiten in Gegenwart von Feuchtigkeit, wie im Mund, sogar verringert sein.

Das Besondere an der aus Parteck® ODT hergestellten Tablettenmatrix ist, dass trotz des Überzugs gleichzeitig bei genügend hohen Härten ein schneller Zerfall der Tabletten im Mund erzielt wird, und dass dieses überraschenderweise auch noch nach einem Beschichtungsprozess unter Einsatz von Wasser- oder Wasser-Alkohol-haltigen Zusammensetzungen der Fall ist, obwohl man eigentlich davon ausgegangen wäre, dass der aufgebrachte Überzug die Zerfallszeit erheblich verlängern würde. In diesem Zusammenhang ist es besonders überraschend, dass je nach der für die Herstellung des Überzugs eingesetzten Zusammensetzung die Zerfallszeit der Tablette bei zunehmender Härte sogar noch verkürzt sein kann und die erhaltenen Tabletten sich als sogenannte IR-Tabletten bzw. FR-Tabletten formulieren lassen, die schnell im Mund zerfallen und direkt die Wirkstoffe über die Mundschleimhaut aufgenommen werden können.

Damit erhält der Pharmazeut, aber auch der Formulierer von tablettenförmigen Nahrungsergänzungsmitteln die Möglichkeit, entsprechende, schnell zerfallende, und mit einem Überzug versehene Tabletten herzustellen. Durch den Überzug lassen sich all die oben aufgezählten Nachteile vermeiden und die entsprechenden Vorteile erzielen.

Für den Hersteller ist insbesondere das Beschichten von Tabletten von Bedeutung, weil dadurch z. B. die enthaltenen Wirkstoffe vor äußeren Einflüssen geschützt werden und insbesondere die Lagerstabilität der, gegebenenfalls in Dosen, bzw. Blistern verpackten Tabletten verbessert werden kann. Es kann aber auch dazu dienen, das äußere Erscheinungsbild der Tabletten für den Anwender zu verbessern und die Wiedererkennbarkeit zu erhöhen.

Entsprechende schnell zerfallend Tabletten sind besonders vorteilhaft für Formulierungen, in denen folgende Wirkstoffe enthalten sein können: Atypical antipsychotics, Antipsychotics, Antidepressants, Anti-histamines, Acetylcholinesterase inhibitors, Analgesics, Anti-pyretics, Anticonvulsant, Anticholinergic, Antiemetics, Benzodiazepines, Corticosteroids, DDC inhibitors [carbidopa], Dopamine receptor antagonists, Monoamine oxidase inhibitors (MAOIs), Nonbenzodiazepine Hypnotics, Opioid analgesic [Tramadol], Proton pump inhibitors, Triptans/Serotonin agonists, NSAIDs, und SSRIs.

Obwohl zu erwarten wäre, dass der Beschichtungsschritt aufgrund der Verwendung von feuchtigkeitshaltigen Zusammensetzung sich auf die Eigenschaften des Tablettenkerns nachteilig auswirkt, hat sich bei Verwendung von Tablettenkernen, die unter Einsatz von Parteck® ODT hergestellt wurden, gezeigt, dass der Beschichtungsprozess durchgeführt werden kann ohne die Zerfallseigenschaften negativ zu beeinflussen. Dieses Ergebnis wurde sowohl für Placebo-Formulierungen als auch für wirkstoffhaltige Tablettenkerne gefunden.

Im Gegensatz dazu zeigten vergleichend geprüfte handelsübliche Fertigmischungen, die ebenfalls einen hohen Mannitgehalt aufweisen, deutlich schlechteres Verhalten während des Beschichtungsvorgangs und in der anschließenden Härte- und Friabilitätsprüfung. So wurde für Vergleichsprodukte ein signifikanter Anstieg der Zerfallszeit von bis zum 10 bis 13-fachen Wert nach dem Beschichtungsprozess gefunden. Dadurch sind diese Tablettenkerne für schnell zerfallende Anwendungen nicht mehr bzw. nur noch beschränkt geeignet. Zusätzlich wurde für einige dieser Produkte während des Beschichtungsvorgangs auch ein großer Abrieb bzw. das Abbrechen von Tablettenbestandteilen (Facette) beobachtet.

Wie oben bereits angesprochen stehen dem Pharmazeuten oder Formulierer zur Herstellung von Tablettenüberzügen in Form von Glasuren oder Filmen im Handel verschiedenste, vorgemischte Zusammensetzungen als Fertiglacksysteme zur Verfügung.

Sie sind vorwiegend für wasserhaltige Coatingsysteme geeignet. In besonderen Fällen handelt es sich um Formulierungen bzw. Coatingsysteme, die auch für Lacke auf auf Wasser-Ethanol-Basis verwendet werden können. Solche Fertiglacksysteme werden von verschiedenen Herstellern angeboten und können problemlos vom Anwender käuflich erworben werden. Je nach gewünschter Verwendung können die Zusammensetzungen, wie bereits angedeutet, zur farblichen Gestaltung der äußeren Erscheinung der überzogenen Tabletten verschiedene stabile Farbpigmente oder wasserlösliche Farbstoffe enthalten, die für den Menschen unbedenklich sind. Als verträgliche Pigmente können z. B. Candurin-Pigmente enthalten sein. Es handelt sich dabei um Pigmente, die als Lebensmittelfarben und als pharmazeutische Hilfsstoffe zugelassen sind und aus natürlichen Silikaten und in der Natur vorkommenden Oxiden, wie Titandioxid und/oder Eisenoxid bestehen, oder um mineralische Perlglanzpigmente. Beim Zusatz der Pigmente ist darauf zu achten, dass dadurch die Eigenschaften des Überzugs möglichst wenig beeinflusst werden. Im vorliegenden Fall darf sich die Löslichkeit bei Kontakt mit Feuchtigkeit, insbesondere mit Speichel nicht verschlechtern, um die rasche Löslichkeit des Tablettenkerns beizubehalten.

In der Zusammensetzung zur Herstellung des Überzug können wasserlöslicher Polymere enthalten sein, ausgewählt aus der Gruppe Carboxymethylcellulose, Carboxyvinylpolymere, hochamylose Stärke, Hydroxyethylcellulose, Hydroxypropylcellulose, Hydroxypropylmethylcellulose (Hypromellose), Methylmethacrylatcopolymere, Polyacrylsäure, Polyvinylalkohol, Polyvinylpyrrolidone, Pullulan, Natriumalginat sowie Mischungen davon.

Bevorzugt werden Überzüge aus Zusammensetzungen hergestellt, die als Filmbildner Polymere ausgewählt aus der Gruppe Polyvinylpyrrolidon, Vinylpyrrolidon / Vinylacetat Copolymer, Polyvinylacetat, Hydroxypropylmethylcellulose und Methacrylestercopolymer oder deren Mischungen enthalten.

Zusammensetzungen zur Herstellung der Filmüberzüge können weiterhin Zucker ausgewählt aus der Gruppe Glucose, Dextrose, Fructose, Laktose, Maltose, Xylose, Sucrose, Maissirup, Sorbit, Hexitol, Maltit, Xylit, Mannit, und deren Mischungen enthalten.
Es kann darin auch mindestens ein Polyalkohol ausgewählt aus der Gruppe Glycerin, Polyethylenglykol (z. B. Makrogol), Propylenglykol, oder deren Mischungen enthalten sein.
Solch eine Zusammensetzung zur Herstellung eines Überzugs auf den Tablettenkernen kann eine essbare und für Lebensmittel geeignete Säure aus der Gruppe Zitronensäure, Äpfelsäure, Weinsäure, Fumarsäure, Phosphorsäure, Oxalsäure und Ascorbinsäure oder Kombinationen dieser Säuren enthalten.
Darüber hinaus kann die filmbildende Zusammensetzung Aromaöle und Geschmacksstoffe enthalten, die sich bereits während des Lösens des äußeren Tablettenüberzugs angenehmen auswirken und einen unangenehmen Geschmack im Mund verhindern. Solche Zusatzstoffe können ausgewählt sein aus der Gruppe Eucolyptol, menthol, Thymol, Verbenon, Rosmarin Verbenone, Eugenol, Geraniol und andere. Es können aber auch entsprechende Mischungen oder andere angenehm schmeckende Zusatzstoffe einschließlich Zucker darin verwendet werden.

Zusätzlich können die Mischungen für die Herstellung der Überzüge weitere Zusätze enthalten, die sich vorteilhaft auf die Filmbildung, die Verarbeitbarkeit und die äußere Erscheinung der fertigen Tablette auswirken.

Entsprechend können Tabletten beispielsweise mit Überzügen versehen sein, die aus Bestandteilen wie Hypromellose, Macrogol 400 [Gemisch von linearen Polymeren mit der allgemeinen Formel H-(OCH₂-CH₂)ₙ-OH, mit mittlerer Molekülmasse von 400], Talkum(Magnesiumsilikathydrat), Titandioxid (E171) und gegebenenfalls Eisen(III)-hydroxid-oxidxH₂O (E172) bestehen.

Wesentlich ist, dass der Tablettenüberzug sich in Gegenwart von Feuchtigkeit, insbesondere von Speichel, in wenigen Sekunden löst. Geeignete Überzüge haben sich in weniger 90 , vorzugsweise weniger als 60 Sekunden gelöst; wobei Überzüge besonders geeignet sind, die sich in einer Zeit von nicht länger als 10 Sekunden gelöst haben. Während des Lösens sollten diese Überzüge kein klebriges Gefühl im Mund erzeugen und angenehm schmecken. Vorzugsweise sollte der Lösevorgang so erfolgen, dass bereits beim Anlösen des Überzugs die Feuchtigkeit in Kontakt kommen kann mit dem darunter liegenden Tablettenkern, sodass sich dieser gleichzeitig mit dem Überzug zu zersetzen beginnt.

Wie bereits vorher angesprochen werden im Handel verschiedenste Fertigmischungen zur Herstellung von Tablettenüberzügen angeboten. Dem Fachmann sind solche Fertigmischungen bekannt. Bei der Wahl einer geeigneten Zusammensetzung stehen für wässrige Zusammensetzungen und solche, in denen neben Wasser als Lösungsmittel auch andere für die Tablettenherstellung geeignete Lösungsmittel zugegeben werden können, zur Verfügung. Vorzugsweise werden Systeme für wässrige Lösungen oder Dispersionen für diesen Zweck verwendet oder solche, die in Wasser lediglich einen verträglichen, leicht flüchtigen Alkohol, wie Ethanol, enthalten. Bei den Fertigmischungen handelt es sich um eine Festsstoffmischung, die vom Anwender in Wasser bzw. einem Wasser-Alkoholhaltigen-Lösungsmittel vor dem Coatingprozess dispergiert bzw. gelöst werden.

In Gegenwart von schnell zerfallenden, feuchtigkeitsempfindlichen Tablettenkernen ist es wesentlich, dass sich mittels der fertigen wasserhaltigen Zusammensetzung für den Überzug in kürzester Zeit ein geschlossener, dünner Schutzfilm an der Oberfläche ausbilden läßt, wobei die Feuchtigkeitskonzentration an der Oberfläche des Kerns möglichst gering gehalten wird. Dieses lässt sich einerseits nur mit niedrigviskosen Zusammensetzungen erreichen, die sich über die Oberfläche ausbreiten, bzw. beim Aufsprühen von geringen Mengen Überzugslösung umgehend aus den auftreffenden Tröpfchen einen Film bilden, andererseits kann die Feuchtigkeitskonzentration an der Oberfläche nur bei erhöhter Temperatur niedrig gehalten werden. Dementsprechend müssen die schnell zerfallenden Tablettenkerne für die Lackierung hohe Anforderungen erfüllen, um auf Grund der hohen mechanischen und thermischen Belastung während des Lackierprozesses nicht beschädigt und/oder zerstört zu werden.

Eine der wesentlichsten Eigenschaften der erfindungsgemäß verwendeten Tablettenkerne besteht darin, dass sie einen geringen Abrieb von < 0,50% aufweisen. Üblicherweise sind für den Fachmann noch Tabletten mit einem Abrieb < 0,2 - 0,3% bedingt für eine Lackierung einsetzbar. Jedoch können zu weiche Tablettenkerne den hohen mechanischen Belastungen in einer Lackiertrommel mit Mischschaufeln nicht standhalten, weil sie zu Beginn aufgrund des fehlenden Schutzes durch eine Lackschicht bereits während der Aufheizzeit beschädigt werden.

Die Versuche zur Herstellung von überzogenen, schnell zerfallenden Tabletten haben darüber hinaus gezeigt, dass zu weiche Tabletten und Tabletten mit hohen Anteilen an Cellulosederivaten, sowie auch ODT-Systeme bereits beim ersten Auftrag des Lacks in einer Menge von 1 - 5 Gew.-% der gesamten Lackauftragsmenge zum Aufquellen neigen. Die eingesetzten Hilfsstoffe in den Tabletten ziehen das Wasser an und die Tabletten beginnen bereits in der Lackiertrommel zu zerfallen. Unter diesen Umständen ist eine weitere Lackierung nicht mehr möglich.

Im Gegensatz dazu haben jetzt Lackierversuche von Tablettenkernen, welche unter Verwendung einer Fertigmischung zur Herstellung von schnell zerfallenden Tabletten, wie sie in WO 2009/152922 A1 hergestellt worden sind, die bisher als allgemeingültig angesehene Aussage widerlegt. Durch Verwendung der gemäß WO 2009/152922 A1 hergestellten Zusammensetzung (Parteck® ODT) kann auf schnell zerfallende Tabletten ein Überzug oder Film unter Verwendung einer wässrigen Zusammensetzung aufgetragen werden ohne dass dieses mit einer signifikanten Verlängerung der Zerfallszeit verbunden ist.

Trotz der aufgetragenen Lackschicht und einem gleichzeitigen Anstieg der Tablettenhärte haben sich die Zerfallszeiten der erhaltenen Filmtabletten im Vergleich zu denen der ursprünglichen Tablettenkerne nur um geringe Nuancen (nicht signifikant) verändert.

Beispielsweise aus Placebo-Formulierungen Tablettenkerne hergestellt und mit einer Fertigmischung als Coating-System (Opadry 200 White, vertrieben von der Fa. Colorcon) behandelt. Es verringerte sich die Zerfallszeit entsprechender Parteck ODT Filmtabletten erstaunlicherweise um 3 Sekunden auf 53 Sekunden im Gegensatz zu der Zerfallszeit der Tablettenkerne, die innerhalb von durchschnittlich 56 Sekunden zerfallen. Das Ergebnis ist überraschend, weil sich die Härte der nicht lackierten Tablettenkerne von 51N auf 108N erhöht nach der Lackierung. Trotz der erheblich höheren Härte kann überraschend eine Abnahme der Zerfallszeit nachgewiesen werden. Bei dem verwendeten Coating-System Opadry White handelt es sich um eine wässrige Zusammensetzung, worin die Filmbildung durch den enthaltenen Polyvinylalkohol erfolgt. Je nach Konzentration kann die Viskosität niedrig eingestellt werden.

Im Vergleich dazu wurden Tabletten unter Verwendung der oben beschriebenen Fertigmischung Ludiflash® hergestellt und unter denselben Bedingungen mit demselben Coating-System (Opadry® 200 White) überzogen. Obwohl es sich bei der verwendeten Fertigmischung ebenfalls um eine Zusammensetzung mit einem hohen Mannitgehalt handelt, steigt die Zerfallszeit in Gegenwart von Feuchtigkeit unerwartet stark an nachdem die Tablettenkerne mit einem Opadry®-Film versehen worden sind. Während die Tablettenkerne eine niedrige Zerfallszeit aufweisen, zeigen die überzogenen Tabletten eine Zunahme der Zerfallszeit um 461 Sekunden bis auf durchschnittlich 501 Sekunden. Auch in diesem Fall ist eine Zunahme der Tablettenhärte zu verzeichnen; und zwar steigt die Härte von 44N der Tablettenkerne auf 93N der Filmtabletten.

Werden Tablettenkerne der beiden Placebo-Formulierungen mit dem Coating-System der Fa. Biogrund (ReadiLycoat) behandelt und überzogen, zeigt sich ein ähnliches Verhalten der beiden unterschiedlichen ODT-Rezepturen.
Bei ReadiLycoat® handelt es sich um ein wässriges Coating-System, worin der Überzug durch ein Stärke-basiertes Polymer gebildet wird.

Die Zerfallszeit der hergestellten Filmtabletten unter Verwendung von Parteck® ODT liegt im Durchschnitt bei 79 Sekunden bei einer Härte von 90N. Sie ist gegenüber der Zerfallszeit der der ursprünglichen Tablettenkerne nur um 23 Sekunden gestiegen und zwar von ursprünglich 56 Sekunden bei einer Härte von 51N.

Die Filmtabletten mit Ludiflash® als Grundlage haben hingegen eine sehr hohe Zerfallszeit von durchschnittlich 400 Sekunden bei einer Härte von 93N. Sie ist somit um 360 Sekunden gestiegen von einer Zerfallszeit der Tablettenkerne von 40 Sekunden bei einer Härte von 44N.

Auch wenn wirkstoffhaltige Tablettenkerne auf der Basis von Parteck® ODT und Ludiflash® hergestellt werden und in entsprechender Weise mit den wässrigen Coating-Systemen behandelt werden, ergibt sich das gleiche Bild. Für diese Versuche wurden Verum-Formulierungen mit einem Anteil von 20% Ascorbinsäure pro Einzeldosis verwendet.

Auch unter diesen Bedingungen ist die Zunahme der Zerfallszeit der erhaltenen Filmtabletten aus Tablettenkernen, in denen Parteck® ODT verwendet worden ist, wesentlich geringer als wenn Ludiflash® zur Herstellung der Tablettenkerne eingesetzt worden ist. Gleichzeitig ist der Anstieg der Härte der lackierten Tabletten, welche Parteck® ODT enthalten, wesentlich höher als wenn Ludiflash® zur Herstellung der Tablettenkerne eingesetzt worden ist. Letztere zeigen nur einen sehr geringer Anstieg der Tablettenhärten nach der Lackierung.

Die Tablettenkerne der Parteck® ODT-Rezeptur haben eine Zerfallszeit von 28 Sekunden, bei einer Härte von 54N. Nach der Lackierung mit dem Coating-System der Fa. Colorcon (Opadry® 200 White) steigt die Zerfallszeit der Filmtabletten um 27 Sekunden auf durchschnittlich 55 Sekunden (Härte von 117N) an und nach der Lackierung mit dem Coating-System der Fa. Biogrund (ReadiLycoat®) ist eine Zerfallszunahme von 46 Sekunden auf 74 Sekunden (Härte von 93N) zu verzeichnen.

Im Gegensatz dazu haben die Tablettenkerne der Ludiflash-Rezeptur eine Zerfallszeit von 22 Sekunden (Härte von 45N), die sich nach der Lackierung drastisch erhöht. Somit haben die Filmtabletten nach der Lackierung mit dem Coating-System der Fa. Colorcon eine Zerfallszeit von 309 Sekunden bei einer Härte von nur 62N, ein Anstieg der Zerfallszeit von 287 Sekunden und die Filmtabletten mit dem Coating-System der Fa. Biogrund haben eine Zerfallszeit von 175 Sekunden bei einer Härte von nur 59N und hier wiederum einen Anstieg der Zerfallszeit von 153 Sekunden.

Darüberhinaus zeigen die Filmtabletten, die unter Verwendung von Parteck® ODT hergestellt worden sind, so gut wie keine Schäden und haben eine ebene und glatte Oberfläche, da die eingesetzten Tablettenkerne die besten Voraussetzungen für eine Lackierung aufweisen. Sie haben eine Härte im Bereich von 50N, um eine schnelle Zerfallszeit zu gewährleisten, weisen aber gleichzeitig eine ausreichende Bruchfestigkeit (mechanische Stabilität) auf, um den Belastungen während der Lackierung, und vor allem während der Aufheizphase, standzuhalten. Die Placebo-Tablettenkerne haben einen Abrieb von 0,37% bei einer durchschnittlichen Härte von 51N und die Verum-Tablettenkerne haben einen Abrieb von 0,45% bei einer durchschnittlichen Härte von 54N.

Die Placebo-Tablettenkerne auf Ludiflash-Basis weisen einen Abrieb von 0,36% auf bei einer Tablettenhärte von durchschnittlich 44N. Dieses sind gute Ausgangsvoraussetzungen für eine Lackierung. Entsprechend weisen die lackierten Tabletten, also die erhaltenen Filmtabletten, eine glatte und ebene Oberfläche auf.

Die Verum-Tablettenkerne auf Ludiflash®-Basis zeigen bei einer durchschnittlichen Härte von 45N jedoch einen sehr hohen Abrieb von 14,71%. Zum Teil deckelten die Tablettenkerne auch während des Abriebtests. Diese schlechten Tabletteneigenschaften haben auch auf die Lackierung einen negativen Einfluss. Die Tabletten können den hohen mechanischen Belastungen während dem Aufheizen nicht standhalten. An vielen Tabletten sind die Kanten am Übergang zum Steg abgebröckelt. Somit verlieren diese Tabletten an Gewicht, und es kann zum Ende der Lackierung keine Bestimmung der Auftragsmenge ermittelt werden, da sich das Einzelgewicht der Filmtabletten im Gegensatz zu den ursprünglichen Tablettenkernen auf Grund der Abbrüche verringert hat. Außerdem weisen die Filmtabletten deutliche Abbrüche an den Seiten auf.

Durch die Versuche wurde gefunden, dass überraschenderweise ODT-Tabletten unter Verwendung von wässrigen Coating-Systemen lackiert werden können. Es wurde dadurch auch gefunden, dass die zur Lackierung verwendbaren Tablettenkerne nicht beliebig zusammengesetzt sein können.

So ist bei herkömmlichen ODT-Systemen, wie beispielsweise unter Verwendung von Ludiflash® zur Herstellung der Tablettenkerne, mit einem signifikanten Anstieg der Zerfallszeit der Filmtabletten zu rechnen im Vergleich zu den ursprünglichen Tablettenkernen. Dieses hat zur Folge, dass eine schnelle Freisetzung des Wirkstoffs bzw. ein schneller Zerfall der Tablette innerhalb der Mundhöhle nicht mehr gegeben ist, wie eigentlich für ODT-Systeme erwünscht und für solche Formulierungen vorgeschrieben ist.

Bei der Verarbeitung, vor allem der Lackierung, von Parteck® ODT ist nur ein geringer Anstieg der Zerfallszeit festzustellen, obwohl eine enorme Zunahme der Härte festzustellen ist im Vergleich zwischen dem ursprünglichen Tablettenkern und der daraus hergestellten Filmtablette. Die Filmtabletten haben trotz der schützenden Lackschicht lediglich Zerfallszeiten von 53 bis 79 Sekunden, sodass sie auch mit Überzug einen schnellen Zerfall und damit auch eine schnelle Freisetzung des Wirkstoffs gewährleistet ist. Ein weiterer Vorteil ist die enorme Festigkeit der Filmtabletten, wodurch sie zusätzlich resistent für mechanische Belastungen sind, die z.B. während der Verpackung und des Transports auftreten.

Aber nicht nur bei Verwendung rein wässriger Coating-Systeme erweist sich die Verwendung von Parteck® ODT als vorteilhaft für die Herstellung von oral schnell zerfallenden Filmtabletten. Auch wenn zur Herstellung von entsprechenden Überzügen Coating-Formulierungen verwendet werden, welche ein Ethanol-Wasser-Gemisch als Lösungsmittel enthält.

Durch die Versuche hat sich gezeigt, dass bei allen Formulierungen unter Verwendung von Parteck® ODT, egal ob es sich um wirkstoffhaltige oder um Placebos handelt, eine überraschende und signifikante Abnahme der Zerfallszeit der Filmtabletten gegenüber der der eingesetzten Tablettenkerne zu beobachten ist, bei gleichzeitiger Zunahme der Härte um 40-75N nach der Lackierung mit einem Coating-System, welches ein Ethanol-Wasser-Gemisch als Lösungsmittel enthält.

Je nach Zusammensetzung des Coating-Systems ist überraschend eine geringe bis starke Abnahme der Zerfallszeit der Tabletten nach dem Aufbringen des Überzugs festzustellen. Die Verringerung der Zerfallszeit schwankt zwischen 1 Sekunde (-4%, im beispielhaften Verarbeitungsbeispiel Nr. 11) bis zu einer Abnahme von 64 Sekunden (-49% in Verarbeitungsbeispielen Nr. 5 und Nr. 6).

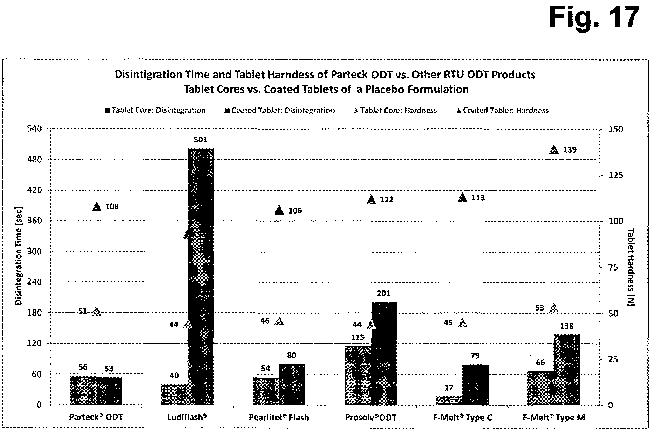

Diese Ergebnisse sind besonders überraschend, da sie sich nicht in gleicher Weise mit vergleichbaren im Handel erhältlichen Fertigmischungen zur Herstellung von schnell zerfallenden Tabletten nachvollziehen lassen. In Fig. 17 sind verschiedene Im Handel erhältliche Mischungen miteinander verglichen, indem einerseits die Zerfallszeiten und die Härten der Tablettenkerne verschiedener Fertigmischungen aufgetragen sind und andererseits die Härten und Zerfallszeiten der entsprechenden mit einem Überzug versehenen Tablettenkerne aufgetragen sind, wobei in jedem Fall ein Überzug mit derselben Zusammensetzung und unter denselben Bedingungen auf die unterschiedlichen Tablettenkerne aufgebracht wurde.

In allen Fällen, in denen die Tablettenkerne aus Fertigmischungen anderer Hersteller bestehen, steigen die Härte und die Zerfallszeit nach dem Aufbringen des Überzugs an. Im Gegensatz dazu nehmen diese Werte für erfindungsgemäße Tablettenkerne ab (hergestellt mit Parteck® ODT.

Beispielsweise wurden unter Verwendung der oben bereits erwähnten vergleichbaren Fertigzusammensetzung Ludiflash ® Vergleichsversuche unter denselben Bedingungen durchgeführt. Hier wird bei allen hergestellten Rezepturen eine Verlängerung der Zerfallszeit der Filmtabletten im Vergleich zu der für die unbehandelten Tablettenkerne ermittelt, und zwar unabhängig davon ob es sich um eine Placebo- oder eine wirkstoffhaltige Tablettenformulierung handelt. Außerdem ist für die wirkstoffhaltige Tabletten-Formulierung so gut wie kein Härteanstieg festzustellen nachdem die Tabletten mit der Lackierung versehen worden sind (45N auf 46N), gleichzeitig steigt aber die Zerfallszeit um durchschnittlich 9 Sekunden.

Dementsprechend ist auch zur Herstellung von schnell zerfallenden Tabletten mit Überzug bei Verwendung einer Ethanol-Wasser-haltigen Coating-Zusammensetzung besonders Parteck® ODT zur Herstellung der Tablettenkerne geeignet.

Die vorliegende Beschreibung ermöglicht es dem Fachmann, die Erfindung umfassend anzuwenden. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, die zitierten Veröffentlichungen und Patentliteratur heranzuziehen. Dementsprechend gelten diese Dokumente als Teil der Offenbarung der vorliegenden Beschreibung. Dieses trifft insbesondere auf die Patentanmeldung WO 2009/152922A1 zu, worin die Herstellung der Fertigmischung Parteck® ODT näher beschrieben ist.

Zum besseren Verständnis der Erfindung werden im Folgenden Beispiele und Vergleichsbeispiele gegeben, die im Rahmen des Schutzbereichs liegen, bzw. die Vorteile der Erfindung verdeutlichen. Diese Beispiele dienen auch zur Veranschaulichung möglicher Varianten. Aufgrund der allgemeinen Gültigkeit des beschriebenen Erfindungsprinzips bzgl. der Verwendung der Tablettenmischungen und der Coating-Systeme sind die gegebenen Beispiele nicht dazu geeignet, den Schutzbereich der vorliegenden Anmeldung nur auf diese zu reduzieren.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew. bzw. mol-% bezogen auf die Gesamtzusammensetzung aufaddieren und nicht darüber hinausgehen können, auch wenn sich aus den angegebenen Prozentbereichen höhere Werte ergeben könnten. Dieses gilt auch für Volumenprozentangaben. Sofern nichts anderes angegeben ist, gelten daher %-Angaben als Gew.-% oder mol-%, mit Ausnahme von Verhältnissen, die in Volumenangaben wiedergegeben sind.

Die in den Beispielen und der Beschreibung sowie in den Ansprüchen gegebenen Temperaturen gelten immer in °C.

### Beispiele

### Verwendete Anlagen und Geräte

1. Rundläuferpresse Korsch PH230 (KORSCH AG, Berlin, Deutschland)
   - 14 Stempelpaare: Ø 11mm, bikonvex, R14.5, Stempel-Nr. 05/11
   - Auswertesystem PMA3 (Pharmapress Measuring and Analysis System) von Korsch
2. Rhönradmischer RRM ELTE 650 von Engelsmann (J. Engelsmann AG, Ludwigshafen, Deutschland)
   - Einsatz 50l Mischfass, ohne Schikanen oder Mischhilfen
3. Lackieranlage O'Hara Labcoat IIX (Ohara Technologies, Richmond Hill, Kanada)
   - Kleine Trommel (d=15 Zoll) bestückt mit einer Düse
   - Zweistoffdüse von Schlick, Durchmesser Düseneinsatz: 0,7mm
4. IKA-Rührwerk (IKA®-Werke GmbH & CO. KG, Staufen, Deutschland)
   - Herstellung der Lacksuspension mit einem Propellerrüher Ø ca. 8cm
5. ERWEKA TBH 30 MD (ERWEKA® GmbH, Heusenstamm, Deutschland)
   - Bestimmung von Höhe, Durchmesser und Härte Inprozess und nach 1 Tag (n=20 Tabletten)
   - aus den 20 Messungen Berechnung der Durchschnittswerte
6. Analysenwaage METTLER AT 201 (Mettler-Toledo GmbH, Giessen, Deutschland)
   - Arbeitsbereich: 0,05 bis 200g
   - Bestimmung des Tabletteneinzelgewichtes nach 1 Tag bzw. Bestimmung des Filmtabletteneinzelgewichtes (n=20 Tabletten bzw. Filmtabletten)
   - aus der Wägung der 20 Tabletten/Filmtabletten Berechnung der Durchschnittswerte
7. Tablettenabrieb am Friabilitätsprüfgerät TA 420 Fa. ERWEKA (ERWEKA® GmbH, Heusenstamm, Deutschland)
   - Geräteparameter und Ausführung der Messungen gemäß Ph.Eur. 7. Ausgabe "Friabiltiät von nicht überzogenen Tabletten" (Benutzung einer Trommel nach Roch)
   - Einwaage bei Abriebtest: Tablettenmasse ≤ 650mg → Gesamtmasse ca. 6,5g, entspricht 17 Tabletten á 400mg = 6,8g
8. 'disi'4 Tabletten Zerfallszeittester: DISI Automatic Disintegration Tester, Fa. Pharmatron Dr. Schleuniger (Pharmatron AG, CH-3600 Thun, Schweiz)
   - Bestimmung der Zerfallszeit der Tabletten nach 1 Tag sowie der Zerfallszeit der Filmtabletten (n=6 Tabletten)
   - Bestimmung in 800ml VE-Wasser bei 37°C ± 2K
   - Geräteparameter und Ausführung gemäß Ph.Eur. 7. Ausgabe "Zerfallszeit von Tabletten und Kapseln", Prüfung A: Tabletten und Kapseln normaler Größe

### Allgemeine Verfahrensbeschreibung der Herstellung

### A. Herstellung der Fertigmischung

Zu tablettierendes Material:
Parteck ODT® (Artikel-Nr.:1.0490.9050, Hersteller: Merck KGaA, Darmstadt, Deutschland)
   oder
Ludiflash® (Artikel-Nr.: 56513304, Hersteller: BASF, Ludwigshafen, Deutschland)
   wird jeweils mit
Ascorbinsäure (Artikel-Nr.: 83568.290, Hersteller: BDH Prolabo chemicals - The Chemical Brand from VWR international, Leuven, Belgien) zusammen in ein 50l Mischfass gegeben und anschließend für 10 Minuten im Rhönradmischer (Geschwindigkeitsstufe 7) homogenisiert.
Auf die erhaltene Mischung wird der Anteil an Magnesiumstearat (Parteck® LUB MST, pflanzlich, Artikel-Nr.: 1.00663.9020, Hersteller: Merck KGaA, Darmstadt, Deutschland) über ein 250µm Laborsieb aufgesiebt und weitere 10 Minuten im Rhönradmischer bei Geschwindigkeitsstufe 7 homogenisiert.

Zur Herstellung der Placebo-Mischung wird das zu tablettierende Material (Parteck® ODT oder. Ludiflash®) direkt zusammen mit dem Anteil an Magnesiumstearat (pflanzlich, Parteck® LUB MST) versetzt und über ein 250µm Laborsieb aufgesiebt. Anschließend werden die Substanzen in ein 50l Mischfass gefüllt und 10 Minuten im Rhönradmischer bei Geschwindigkeitsstufe 7 homogenisiert.

### B. Herstellung der Tabletten

Die vier verschiedenen Mischungen werden auf der Rundläuferpresse PH230 mit unterschiedlichem Druck verpresst. Hierbei ist ist es das Ziel, den Druck so einzustellen, dass eine Tablettenhärte von 50N erreicht wird. Zu diesem Zweck werden während der Tablettierung zu vier Zeitpunkten mindestens 100 Tabletten als Muster zur Auswertung der Preßdaten/galenischen Kennzahlen gezogen.

### C. Lackherstellung

Es werden 90% der vorgesehenen Wassermenge vorgelegt und der Grundlack mit Opadry™ 200 White (Artikel-Nr.: 200F280000, Hersteller:
Colorcon GmbH, Idstein, Deutschland)
   oder
Aqua Polish® Clear (Artikel-Nr.: MY6128, Hersteller: BIOGRUND GmbH, Hünstetten, Deutschland)
   unter Rühren portionsweise zugegeben.
   Der Grundlack wird anschließend gerührt bis ein homogener, geschmeidiger Lack entstanden ist (innerhalb von ca. 30-45 Minuten). In den fertigen Grundlack wurden unter langsamem Rühren die Pigmente eingearbeitet. Bei Vorliegen einer gleichmäßigen Verteilung der Pigmente im Lack ist die Lackherstellung beendet.

### D. Herstellung der Filmtabletten

Die Lackierung der Tablettenkerne wird am O'Hara Labcoat mit der 15" Trommel (kleine Trommel) und einer Düse (Zweistoffdüse Schlick, Düseneinsatz 0,7mm) durchgeführt.
Die vorgegebene Menge an Tablettenkernen wird in die Trommel eingewogen und vor Beginn der Sprühung entstaubt und vorgewärmt. Nach der Aufheizphase wird die entsprechende Lackmenge auf die Tabletten aufgesprüht. Während der gesamten Lackierung wird die Lacksuspension durchgehend gerührt um ein Absetzen der Pigmente zu verhindern. Darüber hinaus werden jeweils nach einer Lackauftragsmenge von 10, 20, 30, 40, 50, 60, 70, 80, 90 und 100% Muster zur späteren visuellen Bewertung und Auswertung der physikalischen Eigenschaften gezogen. Im Anschluss an die Sprühung werden die Filmtabletten in der Trommel bis zu einer bestimmten Ablufttemperatur getrocknet und danach in Doppel-PE-Beutel und in eine Weißblechdose verpackt.

Während des Herstellprozesses werden die folgenden Parameter kontrolliert und protokolliert:
1. Zu- und Ablufttemperatur in °C während des Aufheizens
2. Ansatzgröße (Einsatzmenge FITABK und Lackauftragsmenge in g)
3. Zu- und Ablufttemperatur in °C während dem Sprühvorgang
4. Sprührate in g/min
5. Sprühdruck in bar
6. Düsendurchmesser in mm
7. Zu- und Ablufttemperatur in °C bei der Trocknung

### E. Prozessparameter für das Herstellen und Coating der Tablettenkerne

| Parameter | Einstellung/Wert |
|---|---|
| Herstellung der Fertigmischung | |
| Maschenweite Handsieb für Schmiermittel | 250 µm (Magnesiumstearat) |
| Mischzeit | 10 min für Placebo-Formulierungen 2 x 10 min für Verum-Formulierungen |
| Mischgeschwindigkeit | 7 UpM |

| Tablettierung | |
|---|---|
| Maschinenleistung | 50 UpM |
| Preßkraft | 5,1 - 14,5 kN* |
| Fülltiefe | 5,6 - 6,5 mm* |
| Steghöhe | 1,7 - 2,6 mm* |
| Tablettenhärte Inprozess | 40 - 55 N |
| | * je nach ODT-System und Formulierung |

| Lackierung (Filmcoating) | |
|---|---|
| Düsendurchmesser | 0,7 mm |
| Abstand Düse-Kernbett | ca. 15 cm |
| Flachstrahlluft | 0,5 bar |
| Zerstäuberluft | 1 bar |
| Innendurchmesser Sprühschlauch | 3,2 mm |
| Zuluftmenge | 580 - 600 m³/h |
| Trommeldrehzahl Aufheizen | Tastbetrieb |
| Trommeldrehzahl Sprühen | 19 - 20 UpM |
| Trommeldrehzahl Trocknung | 3 - 5 UpM |
| Zulufttemperatur Aufheizen (Soll) | 55 °C (+/- 3K) |
| Zulufttemperatur Sprühen (Ist) | 54 - 63 °C |
| Zulufttemperatur Trocknung (Soll) | 65 C (+/- 3K) |
| Ablufttemperatur Beginn Sprühen (Ist) | 50 - 58 °C |
| Ablufttemperatur Sprühen (Ist) | 46 - 51 °C |
| Trocknung bis Ablufttemperatur (Ist) | 59 - 60 °C |
| Sprühzeit | 50 min |
| Sprührate | 11 - 14 g/min |
| Trockenzeit | 10 min |

### Zusammensetzungen der Fertigmischung und der Filmtablettenkerne

### a. Placebo-Formulierung enthaltend Parteck® ODT (Fa. Merck):

### Formulierung enthaltend Parteck® ODT und 1% Parteck® LUB MST:

| **Pos.** | **Material-Nr.** | **Material-Bezeichnung** | **Anteil in %** | **Ansatz in 9** | **EZD* in mg** |
|---|---|---|---|---|---|
| **1** | 1.00490.9050 | Parteck® ODT | 99 | 11.880 | 396 |
| **2** | 1.00663.9020 | Parteck® LUB MST (Mg.-stearat) | 1 | 120 | 4 |
| | | | **100** | **12.000** | **400** |

| | | | | | |
|---|---|---|---|---|---|
| * Pos. = Position; ** EZD = Einzeldosis Filmtablettenkern Placebo-Formulierung enthaltend Ludiflash®: | | | | | |

### Formulierung enthaltend Ludiflash® + 1% Parteck® LUB MST

| **Pos.** | **Material-Nr.** | **Material-Bezeichnung** | **Anteil in %** | **Ansatz in 9** | **EZD in mg** |
|---|---|---|---|---|---|
| **1** | 56513304 | Ludiflash® (BASF) | 99 | 4.950 | 396 |
| **2** | 1.00663.9020 | Parteck® LUB MST (Mg.-stearat) | 1 | 50 | 4 |
| | | | **100** | **5.000** | **400** |

Parteck® ODT (Fa. Merck) Verum-Formulierung

### Formulierung Tablettenkerne enthaltend Parteck® ODT + 20% Ascorbinsäure + 1% Parteck® LUB MST

| **Pos.** | **Material-Nr.** | **Material-Bezeichnung** | **Anteil in %** | **Ansatz in g** | **EZD in mg** |
|---|---|---|---|---|---|
| **1** | 1.00490.9050 | Parteck® ODT | 79 | 5.135 | 316 |
| **2** | 83568.290 | L (+) Ascorbinsäure | 20 | 1.300 | 80 |
| **3** | 1.00663.9020 | Parteck® LUB MST (Mg.-stearat) | 1 | 65 | 4 |
| | | | **100** | **6.500** | **400** |

### b. Ludiflash® (Fa. BASF) Verum-Formulierung

### Formulierung Tablettenkerne enthaltend Ludiflash® + 20% Ascorbinsäure + 1% Parteck® LUB MST

| **Pos.** | **Material-Nr.** | **Material-Bezeichnung** | **Anteil in %** | **Ansatz in g** | **EZD in mg** |
|---|---|---|---|---|---|
| **1** | 56513304 | Ludiflash® (BASF) | 79 | 3.950 | 316 |
| **2** | 83568.290 | L (+) Ascorbinsäure | 20 | 1.000 | 80 |
| **3** | 1.00663.9020 | Parteck® LUB MST (Mg.-stearat) | 1 | 50 | 4 |
| | | | **100** | **5.000** | **400** |

In **Fig. 17** sind die Härten und Zerfallszeiten miteinander verglichen, und zwar von Tablettenkernen und Filmtabletten, welche unter Verwendung von verschiedenen, im Markt erhältlichen Vormischungen, welche unterschiedliche Mannitqualitäten verschiedener Anbieter enthaltenden hergestellt worden sind.

### Zusammensetzung Lack und Filmtabletten - Übersicht

### a) Inhaltsstoffe der eingesetzten Fertiglack-Produkte der Firma Colcorcon und der Firma Biogrund

**Übersicht der Inhaltsstoffe Opardy™ 200 White (Fa. Colorcon)**

| **Opardy™ 200 White (Fa. Colorcon)** |
|---|
| Polyvinylalkohol (PVA) |
| Titandioxid |
| Talkum |
| Polyethylenglycol (Macrogol) |
| Methacrylsäure-Copolymer |
| Natriumbicarbonat |

**Übersicht der Inhaltsstoffe Aqua Polish® Clear (Fa. Biogrund)**

| **Aqua Polish® Clear (Fa. Biogrund)** |
|---|
| Hydroxypropylmethylcellulose |
| Hydroxypropylcellulose |
| Talkum |
| Modifizierte Stärke |
| Miglyol |

Die Wassermenge wurde bei der Berechnung der Einzeldosis der Filmtablette nicht berücksichtigt.

### b) Grundlage Opadry 200 White (Fa. Colorcon) + Colorona Majestic Green (Fa. Merck)

**Placebo-Formulierung Filmtabletten für Verarbeitungsbeispiele Nr. 1 und Vergleichsbeispiel Nr. 1**

| **Pos.** | **Material-Nr.** | **Material-Bezeichnung** | **Ansatz in g** | **Ansatz in g + 10%** | **EZD in mg** |
|---|---|---|---|---|---|
| **1** | X | FITAB-Kerne Placebo | 2.000 | X | 400,0 |
| **2** | 200F280000 | Opadry™ 200 White | 44 | 48,40 | 8,8 |
| **3** | 1.17190.1000 | Colorona® Majestic Green | 6 | 6,60 | 1,2 |
| **4** | 2.00001.0000 | Aqua Purificata | 550 | 605 | 110,0 |
| | | | **2.600** | **660** | **410,0** |

### c) Grundlage Aqua Polish® (Fa. Biogrund) + Candurin® Brown Amber (Fa. Merck)

**Placebo-Formulierung Filmtabletten für Verarbeitungsbeispiel Nr. 2 und Vergleichsbeispiel Nr. 2**

| **Pos.** | **Material-Nr.** | **Material-Bezeichnung** | **Ansatz in g** | **Ansatz in g + 10%** | **EZD in mg** |
|---|---|---|---|---|---|
| **1** | X | FITAB-Kerne Placebo | 2.000 | X | 400,0 |
| **2** | MY6128 | Aqua Polish® Clear | 44 | 48,40 | 8,8 |
| **3** | 1.20617.1000 | Candurin® Brown Amber | 6 | 6,60 | 1,2 |
| **4** | 2.00001.0000 | Aqua Purificata | 550 | 605 | 110,0 |
| | | | **2.600** | **660** | **410,0** |

### d) Grundlage Opadry 200 White (Fa. Colorcon) + Candurin Red Lustre (Fa. Merck)

**Verum-Formulierung Filmtabletten für Verarbeitungsbeispiele Nr. 3 und Vergleichsbeispiel Nr. 3**

| **Pos.** | **Material-Nr.** | **Material-Bezeichnung** | **Ansatz in g** | **Ansatz in g + 10%** | **EZD in mg** |
|---|---|---|---|---|---|
| **1** | X | FITAB-Kerne Verum | 2.000 | X | 400,0 |
| **2** | 200F280000 | Opadry™ 200 White | 44 | 48,40 | 8,8 |
| **3** | 1.20619.1000 | Candurin® Red Lustre | 6 | 6,60 | 1,2 |
| **4** | 2.00001.0000 | Aqua Purificata | 550 | 605 | 110,0 |
| | | | **2.600** | **660** | **410,0** |

### e) Grundlage Aqua Polish® (Fa. Biogrund) + Candurin® Brown Amber (Fa. Merck)

**Verum-Formulierung Filmtabletten für Verarbeitungsbeispiele Nr. 4 und Vergleichsbeispiel Nr. 4**

| **Pos.** | **Material-Nr.** | **Material-Bezeichnung** | **Ansatz in g** | **Ansatz in g + 10%** | **EZD in mg** |
|---|---|---|---|---|---|
| **1** | X | FITAB-Kerne Verum | 2.000 | X | 400,0 |
| **2** | MY6128 | Aqua Polish® Clear | 44 | 48,40 | 8,8 |
| **3** | 1.20617.1000 | Candurin® Brown Amber | 6 | 6,60 | 1,2 |
| **4** | 2.00001.0000 | Aqua Purificata | 550 | 605 | 110,0 |
| | | | **2.600** | **660** | **410,0** |

Die Auftragsmenge lag theoretisch bei allen Coatingversuchen pro Ansatz bei ca. 600g, und dies entspricht ca. 2,4% bezogen auf die EZD. Bei dieser Berechnung wurde wiederum die Wassermenge nicht berücksichtigt.

### Zusammensetzung und Herstellparameter der einzelnen Verarbeitungsbeispiele

### a. Ansatzgröße der Verarbeitungsbeispiele Nr. 1 bis Nr. 4

Für die im Folgenden aufgeführten Verarbeitungsbeispiele wurden jeweils eine Einsatzmenge von 2000g an Filmtablettenkernen in die Lackiertrommel vorgelegt und eine Lackmenge von 595g aufgesprüht.

### b. Formulierung Verarbeitungsbeispiel Nr. 1

Beschichtung einer Placebo-Tablette (Parteck ODT) mit einem üblichen Fertiglack Opadry 200 White (Hersteller Colorcon GmbH, D-65510 Idstein, Deutschland) plus Farbpigment Candurin Majestic Green (Hersteller Merck KGaA, D-64293 Darmstadt, Deutschland)

**Gesamtübersicht der Formulierung für Verarbeitungsbeispiel Nr. 1**

| **Pos.** | **Material-Bezeichnung** | **Anteil in %** | **EZD in mg** |
|---|---|---|---|
| **1** | Parteck® ODT | 96,6 | 396 |
| **2** | Parteck® LUB MST (Mg.-stearat) | 1,0 | 4 |
| **3** | Opadry™ 200 White | 2,1 | 8,8 |
| **4** | Colorona® Majestic Green | 0,3 | 1,2 |
| **5** | Aqua Purificata | X | 110,0 |
| | | **100** | **410** |

Aus den Versuchsreihen werden im Folgenden die Versuchsergebnisse von unter gleichen Bedingungen hergestellten Tabletten aus den Fertigmischungen Parteck® ODT und Ludiflash® und deren Verhalten bei Lackierung mit unterschiedlichen Caoting-Systemen miteinander verglichen. Diese Versuchsergebnisse sind beispielhaft und zeigen das überraschende vorteilhafte Verhalten von Filmtabletten, die bei Verwendung von Parteck® ODT zur Herstellung der Tablettenkerne erhalten werden.

**Tabelle 1: Übersicht der Arbeitsbeispiele**

| Beisp. Nr. | Zusammensetzung |
|---|---|
| Verarbeitungsbeispiele | |
| Nr. 1 | Beschichtung einer Placebo-Tablette (Parteck® ODT) mit einem üblichen Fertiglack Opadry® 200 White (Hersteller Colorcon GmbH, D-65510 Idstein, Deutschland) plus Farbpigment Candurin® Majestic Green (Hersteller Merck KGaA, D-64293 Darmstadt, Deutschland) |
| Nr. 2 | Beschichtung einer Placebo-Tablette (Parteck® ODT) mit einem üblichen Fertiglack Aqua Polish® Clear (Hersteller Biogrund GmbH, D-65510 Hünstetten, Deutschland) plus Farbpigment Candurin® Brown Amber (Hersteller Merck KGaA, D-64293 Darmstadt, Deutschland) |
| Nr. 3 | Beschichtung einer Verum-Tablette mit Ascorbinsäure als Modell-Wirkstoff (Parteck® ODT) mit einem üblichen Fertiglack Opadry® 200 White (Hersteller Colorcon GmbH, D-65510 Idstein, Deutschland) plus Farbpigment Candurin® Red Lustre (Hersteller Merck KGaA, D-64293 Darmstadt, Deutschland) |
| Nr. 4 | Beschichtung einer Verum-Tablette mit Ascorbinsäure als Modell-Wirkstoff (Parteck® ODT) mit einem üblichen Fertiglack Aqua Polish® Clear (Hersteller Biogrund GmbH, D-65510 Hünstetten, Deutschland) plus Farbpigment Candurin® Red Lustre (Hersteller Merck KGaA, D-64293 Darmstadt, Deutschland) |
| Nr. 5 | Beschichtung einer Placebo-Tablette (Parteck® ODT) mit einem 5%igen Anteil an Fertiglack ReadiLycoat® (Hersteller Biogrund GmbH, D-65510 Hünstetten, Deutschland) |
| Nr. 6 | Beschichtung einer Placebo-Tablette (Parteck® ODT) mit einem 5%igen Anteil an Fertiglack ReadiLycoat® (Hersteller Biogrund GmbH, D-65510 Hünstetten, Deutschland) plus 4% Farbpiqment Candurin® Silver Lustre (Hersteller Merck KGaA, D-64293 Darmstadt, Deutschland) |
| Nr. 7 | Beschichtung einer Placebo-Tablette (Parteck® ODT) mit einem 5%igen Anteil an Fertiglack ReadiLycoat® (Hersteller Biogrund GmbH, D-65510 Hünstetten, Deutschland) plus 0,015% Farbstoff Blau E133 |
| Nr. 8 | Beschichtung einer Placebo-Tablette (Parteck® ODT) mit einem 5%igen Anteil an Fertiglack ReadiLycoat® (Hersteller Biogrund GmbH, D-65510 Hünstetten, Deutschland) plus 1% Farbpigment Candurin® Silver Lustre (Hersteller Merck KGaA, D-64293 Darmstadt, Deutschland) und 0,015% Farbstoff Blau E133 |
| Nr. 9 | Beschichtung einer Placebo-Tablette (Parteck® ODT) mit einem 5%igen Anteil an Fertiglack ReadiLycoat® (Hersteller Biogrund GmbH, D-65510 Hünstetten, Deutschland) plus 4% Farbpigment Candurin® Silver Lustre (Hersteller Merck KGaA, D-64293 Darmstadt, Deutschland) und 0,015% Farbstoff Blau E133 |
| Nr. 10 | analog Verarbeitungsbeispiel-Nr. 5 |
| Nr. 11 | Beschichtung einer Verum-Tablette (Parteck® ODT) mit einem 5%igen Anteil an Fertiglack ReadiLycoat ® (Hersteller Biogrund GmbH, D-65510 Hünstetten, Deutschland) |

### Vergleichsbeispiele

| Beisp. Nr. | Zusammensetzung |
|---|---|
| Vergleichsbeispiele | |
| Nr. 1 | Beschichtung einer Placebo-Tablette (Mitbewerberprodukt, Ludiflash®, BASF) mit einem üblichen Fertiglack Opadry® 200 White (Hersteller Colorcon GmbH, D-65510 Idstein, Deutschland) plus Farbpigment Candurin® Majestic Green (Hersteller Merck KGaA, D-64293 Darmstadt, Deutschland) |
| Nr. 2 | Beschichtung einer Placebo-Tablette (Mitbewerberprodukt, Ludiflash®, BASF) mit einem üblichen Fertiglack Aqua Polish® Clear (Hersteller Biogrund GmbH, D-65510 Hünstetten, Deutschland) plus Farbpigment Candurin® Brown Amber (Hersteller Merck KGaA, D-64293 Darmstadt, Deutschland) |
| Nr. 3 | Beschichtung einer Verum-Tablette mit Ascorbinsäure als Modell-Wirkstoff (Mitbewerberprodukt, Ludiflash®, BASF) mit einem üblichen Fertiglack Opadry® 200 White (Hersteller Colorcon GmbH, D-65510 Idstein, Deutschland) plus Farbpigment Candurin® Red Lustre (Hersteller Merck KGaA, D-64293 Darmstadt, Deutschland) |
| Nr. 4 | Beschichtung einer Verum-Tablette mit Ascorbinsäure als Modell-Wirkstoff (Mitbewerberprodukt, Ludiflash®, BASF) mit einem üblichen Fertiglack Aqua Polish® Clear (Hersteller Biogrund GmbH, D-65510 Hünstetten, Deutschland) plus Farbpigment Candurin® Red Lustre (Hersteller Merck KGaA, D-64293 Darmstadt, Deutschland) |
| Nr. 5 | Beschichtung einer Placebo-Tablette (Mitbewerberprodukt, Ludiflash®, BASF) mit einem 5%igen Anteil an Fertiglack ReadiLycoat® (Hersteller Biogrund GmbH, D-65510 Hünstetten, Deutschland) |
| Nr. 6 | Beschichtung einer Verum-Tablette (Mitbewerberprodukt, Ludiflash®, BASF) mit einem 5%igen Anteil an Fertiglack ReadiLycoat® (Hersteller Biogrund GmbH, D-65510 Hünstetten, Deutschland) |

Vergleich der Daten Parteck®ODT (Fa. Merck) / Ludiflash® (Fa. BASF) - Lackierung auf wässriger Basis

### Erläuterung zu den Abkürzungen:

FITAB = Filmtablette
TABLTK = Tablettenkern (Filmtablettenkern)

### Vergleich der Placebo-Rezepturen - Lackierung mit Opadry®200:

Verarbeitungsbeispiele Nr. 1 und Vergleichsbeispiel Nr. 1

### Verarbeitungsbeispiel Nr. 1:

Beschichtung einer Placebo-Tablette (Parteck® ODT) mit einem üblichen Fertiglack Opadry® 200 White (Hersteller Colorcon GmbH, D-65510 Idstein, Deutschland) plus Farbpigment Candurin® Majestic Green (Hersteller Merck KGaA, D-64293 Darmstadt, Deutschland)

### Vergleichsbeispiel Nr. 1:

Beschichtung einer Placebo-Tablette (Ludiflash®, BASF) mit einem üblichen Fertiglack Opadry® 200 White (Hersteller Colorcon GmbH, D-65510 Idstein, Deutschland) plus Farbpigment Candurin® Majestic Green (Hersteller Merck KGaA, D-64293 Darmstadt, Deutschland)

**Tabelle 2: Vergleich Verarbeitungsbeispiel Nr. 1 und Vergleichsbeispiel Nr. 1**

| | **Verarbeitungsbeispiel Nr. 1** | **Vergleichsbeispiel Nr. 1** |
|---|---|---|
| Parameter | Parteck® ODT + 1% Parteck® LUB MST + Opadry™ 200 + Colorona® Majestic Green | Ludiflash® + 1% Parteck® LUB MST + Opadry™ 200 + Colorona® Majestic Green |
| TABLTK Härte n. 1 Tag [N] | 51 | 44 |
| s.rel in % | 7,89 | 7,31 |
| FITAB Härte n. 1 Tag [N] | 108 (+112%) | 93 (+111%) |
| s.rel in % | 11,58 | 10,45 |
| Tablettengewicht [mg] | 399,95 | 407,83 |
| s.rel in % | 0,65 | 0,55 |
| Filmtablettengewicht [mg] | 405,40 | 412,90 |
| s.rel in % | 2,37 | 11,16 |
| Lackauftragsmenge [mg] | 5,45 | 5,07 |
| Zerfallszeit, 37°C TABLTK [sek] | 56 | 40 |
| Zerfallszeit, 37°C FITAB [sek] | 53 (-5%) | 501 (+1153%) |
| Friabilität TABLTK [%] | 0,37 | 0,36 |
| Friabilität FITAB [%] | 0,00 | 0,00 |

### 1.1.1. Vergleich Aussehen der Filmtabletten Verarbeitungsbeispiel Nr. 1 mit Vergleichsbeispiel Nr. 1

### 1.1.2. Vergleich der Eigenschaften Härte und Zerfallszeit des Verarbeitungsbeispiel Nr. 1 mit Vergleichsbeispiel Nr. 1

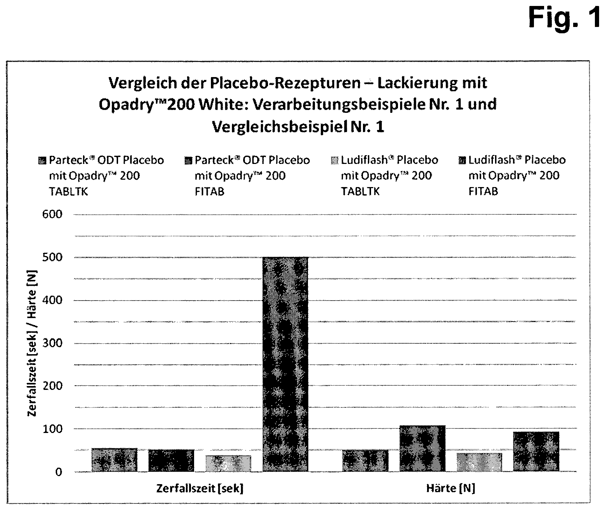

**Fig. 1****:** Darstellung der Auswertung des Vergleichs der Härten und der Zerfallszeiten vor und nach der Herstellung des Überzugs des Verarbeitungsbeispiels Nr. 1 und Vergleichsbeispiel Nr. 1

Die Parteck® ODT Placbeo Tablettenkerne haben bei einer Härte von 51 N eine Zerfallszeit von 56 Sekunden, ähnliche Eigenschaften zeigen auch die Ludiflash® Placebo Tablettenkerne mit einer Zerfallszeit von 40 Sekunden bei einer Tablettenhärte von 44N.
Nach der Lackierung mit Opadry™ 200 White sind allerdings erhebliche Unterschiede festzustellen. So steigt die Härte der Parteck® ODT Placebo Filmtabletten auf 108N und die Zerfallszeit beträgt trotzdem nur 53 Sekunden. Im Gegenzug steigt die Zerfallszeit der Ludiflash® Placebo Filmtabletten auf 501 Sekunden bei einer Härte von 93N.

### 1.2. Vergleich der Placebo-Rezepturen - Lackierung mit Aqua Polish® Clear: Verarbeitungsbeispiele Nr. 2 und Vergleichsbeispiel Nr. 2

### Verarbeitungsbeispiel Nr. 2:

Beschichtung einer Placebo-Tablette (Parteck ODT) mit einem üblichen Fertiglack Aqua Polish Clear (Hersteller Biogrund GmbH, D-65510 Hünstetten, Deutschland) plus Farbpigment Candurin Brown Amber (Hersteller Merck KGaA, D-64293 Darmstadt, Deutschland)

### Vergleichsbeispiel Nr. 2:

Beschichtung einer Placebo-Tablette (Mitbewerberprodukt, Ludiflash, BASF) mit einem üblichen Fertiglack Aqua Polish Clear (Hersteller Biogrund GmbH, D-65510 Hünstetten, Deutschland) plus Farbpigment Candurin Brown Amber (Hersteller Merck KGaA, D-64293 Darmstadt, Deutschland)

**Tabelle 3: Vergleich Verarbeitungsbeispiele Nr. 2 und Vergleichsbeispiel Nr. 2**

| Parameter | Parteck® ODT + 1% Parteck® LUB MST + Aqua Polish® Clear + Candurin® Brown Amber | Ludiflash® + 1% Parteck® LUB MST + Aqua Polish® Clear + Candurin® Brown Amber |
|---|---|---|
| TABLTK Härte n. 1 Tag [N] | 51 | 44 |
| s.rel in % | 7,89 | 7,31 |
| FITAB Härte n. 1 Tag [N] | 90 (+77%) | 93 (+111%) |
| s.rel in % | 9,09 | 8,05 |
| Tablettengewicht [mg] | 399,95 | 407,83 |
| s.rel in % | 0,65 | 0,55 |
| Filmtablettengewicht [mg] | 407,50 | 416,40 |
| s.rel in % | 3,30 | 1,68 |
| Lackauftragsmenge [mg] | 7,55 | 8,75 |
| Zerfallszeit, 37°C TABLTK [sek] | 56 | 40 |
| Zerfallszeit, 37°C FITAB [sek] | 79 (+41%) | 400 (+900%) |
| Friabilität TABLTK [%] | 0,37 | 0,36 |
| Friabilität FITAB [%] | 0,00 | 0,00 |

### Vergleich Aussehen der Filmtabletten Verarbeitungsbeispiel Nr. 2 mit Vergleichsbeispiel Nr. 2

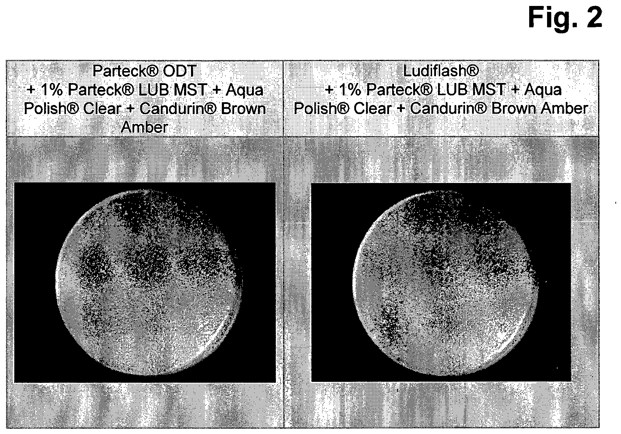

**Fig. 2****:** Vergleich Aussehen FITAB Parteck® ODT Placebo und Ludiflash® Placebo lackiert mit Aqua Polish® Clear

### 1.2.1. Fazit des Vergleichs Verarbeitungsbeispiel Nr. 2 mit Vergleichsbeispiel Nr. 2

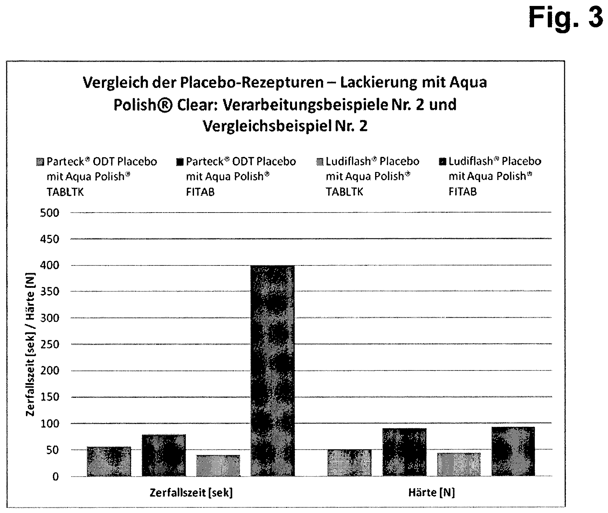

**Fig. 3****:** Darstellung der Auswertung des Vergleichs Verarbeitungsbeispiel Nr. 2 und Vergleichsbeispiel Nr. 2

Die Parteck® ODT Placbeo Tablettenkerne haben bei einer Härte von 51 N eine Zerfallszeit von 56 Sekunden, ähnliche Eigenschaften zeigen auch die Ludiflash® Placebo Tablettenkerne mit einer Zerfallszeit von 40 Sekunden bei einer Tablettenhärte von 44N.

Nach der Lackierung mit Opadry™ 200 White sind allerdings signifikante Unterschiede festzustellen. So steigt die Härte der Parteck® ODT Placebo Filmtabletten auf 90N (+77%) und die Zerfallszeit beträgt nur 79 Sekunden (+41%). Im Gegenzug steigt die Zerfallszeit der Ludiflash® Placebo Filmtabletten auf 400 Sekunden (+900%) bei einer Härte von 93N (+111%).

### 1.3. Vergleich der wirkstoffhaltigen-Rezepturen - Lackierung mit Opadry™ 200: Verarbeitungsbeispiele Nr. 3 und Vergleichsbeispiel Nr. 3

### Verarbeitungsbeispiel Nr. 3:

Beschichtung einer Verum-Tablette mit Ascorbinsäure als Modell-Wirkstoff (Parteck ODT) mit einem üblichen Fertiglack Opadry 200 White (Hersteller Colorcon GmbH, D-65510 Idstein, Deutschland) plus Farbpigment Candurin Red Lustre (Hersteller Merck KGaA, D-64293 Darmstadt, Deutschland)

### Vergleichsbeispiel Nr. 3:

Beschichtung einer Verum-Tablette mit Ascorbinsäure als Modell-Wirkstoff (Mitbewerberprodukt, Ludiflash, BASF) mit einem üblichen Fertiglack Opadry 200 White (Hersteller Colorcon GmbH, D-65510 Idstein, Deutschland) plus Farbpigment Candurin Red Lustre (Hersteller Merck KGaA, D-64293 Darmstadt, Deutschland)

**Tabelle 4: Vergleich Verarbeitungsbeispiele Nr. 3 und Vergleichsbeispiel Nr. 3**

| Parameter | Parteck® ODT + 20% Ascorbinsäure + 1% Parteck® LUB MST +Opadry™ 200 + Candurin® Red Lustre | Ludiflash® + 20% Ascorbinsäure + 1% Parteck® LUB MST + Opadry™ 200 + Candurin® Red Lustre |
|---|---|---|
| TABLTK Härte n. 1 Tag [N] | 54 | 45 |
| s.rel in % | 7,14 | 10,24 |
| FITAB Härte n. 1 Tag[N] | 117 (+117%) | 62 (+38%) |
| s.rel in % | 10,55 | 10,26 |
| Tablettengewicht [mg] | 408,60 | 403,58 |
| s.rel in % | 0,58 | 0,60 |
| Filmtablettenqewicht [mg] | 417,10 | 408,00 |
| s.rel in % | 3,55 | 3,17 |
| Lackauftragsmenge [mg] | 8,50 | 4,42 |
| Zerfallszeit, 37°C TABLTK [sek] | 28 | 22 |
| Zerfallszeit, 37°C FITAB [sek] | 55 (+96%) | 309 (+1305%) |
| Friabilität TABLTK [%] | 0,45 | 14,71 |
| Friabilität FITAB [%] | 0,00 | 0,00 |

### 1.3.1. Vergleich Aussehen der Filmtabletten Verarbeitungsbeispiel Nr. 3 mit Vergleichsbeispiel Nr. 3

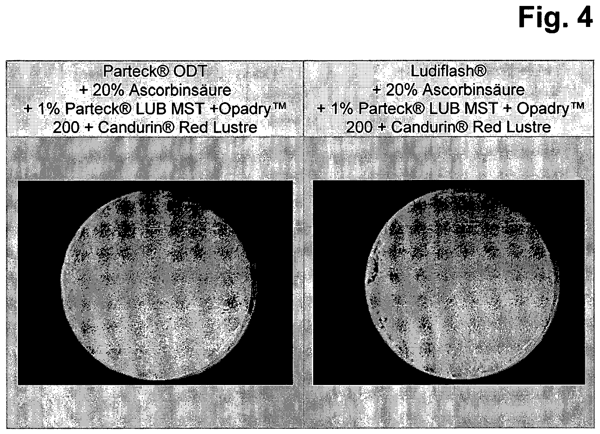

**Fig. 4****:** Vergleich Aussehen FITAB Parteck® ODT Verum und Ludiflash® Verum lackiert mit Opadry™ 200

Während die aus Parteck® ODT hergestellte Filmtablette einen relativ gleichmäßigen Rand aufweist, zeigt die aus Ludiflash hergestellte Filmtablette erhebliche Abbrüche am Rand.

### 1.3.2. Fazit des Vergleichs Verarbeitungsbeispiel Nr. 2 mit Vergleichsbeispiel Nr. 2

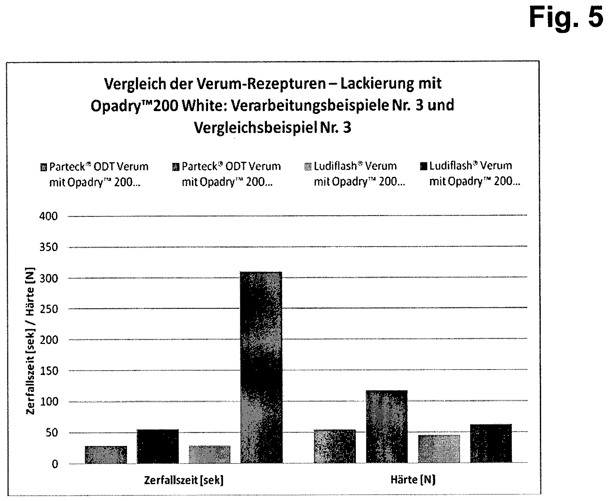

**Fig. 5****:** Darstellung der Auswertung des Vergleichs Verarbeitungsbeispiel Nr. 3 und Vergleichsbeispiel Nr. 3

Die Parteck® ODT Placebo Tablettenkerne haben bei einer Härte von 54N eine Zerfallszeit von 28 Sekunden, ähnliche Eigenschaften zeigen auch die Ludiflash® Placebo Tablettenkerne mit einer Zerfallszeit von 22 Sekunden bei einer Tablettenhärte von 45N.

Nach der Lackierung mit Opadry™ 200 White sind allerdings signifikante Unterschiede festzustellen. So steigt die Härte der Parteck® ODT Placebo Filmtabletten auf 117N (+117%) und die Zerfallszeit beträgt trotzdem nur 55 Sekunden (+96%). Im Gegenzug steigt die Zerfallszeit der Ludiflash® Placebo Filmtabletten auf 309 Sekunden (+1305%) bei einer Härte von 62N (+38%).

### 1.4. Vergleich der Verum-Rezepturen - Lackierung mit Aqua Polish® Clear: Verarbeitungsbeispiele Nr. 4 und Vergleichsbeispiel Nr. 4

### Verarbeitungsbeispiel Nr. 4:

Beschichtung einer Verum-Tablette mit Ascorbinsäure als Modell-Wirkstoff (Parteck ODT) mit einem üblichen Fertiglack Aqua Polish Clear (Hersteller Biogrund GmbH, D-65510 Hünstetten, Deutschland) plus Farbpigment Candurin Red Lustre (Hersteller Merck KGaA, D-64293 Darmstadt, Deutschland)

### Vergleichsbeispiel Nr. 4:

Beschichtung einer Verum-Tablette mit Ascorbinsäure als Modell-Wirkstoff (Mitbewerberprodukt, Ludiflash) mit einem üblichen Fertiglack Aqua Polish Clear (Hersteller Biogrund GmbH, D-65510 Hünstetten, Deutschland) plus Farbpigment Candurin Red Lustre (Hersteller Merck KGaA, D-64293 Darmstadt, Deutschland)

**Tabelle 5: Vergleich Verarbeitungsbeispiele Nr. 4 und Vergleichsbeispiel Nr. 4**

| Parameter | Parteck® ODT + 20% Ascorbinsäure + 1% Parteck® LUB MST + Aqua Polish® Clear + Candurin® Red Lustre | Ludiflash® + 20% Ascorbinsäure + 1% Parteck® LUB MST + Aqua Polish® Clear + Candurin® Red Lustre |
|---|---|---|
| TABLTK Härte n. 1 Tag [N] | 54 | 45 |
| s.rel in % | 7,14 | 10,24 |
| FITAB Härte n. 1 Tag [N] | 93 (+72%) | 59 (+31%) |
| s.rel in % | 4,70 | 10,88 |
| Tablettengewicht [mg] | 408,60 | 403,58 |
| s.rel in % | 0,58 | 0,60 |
| Filmtablettengewicht [mg] | 417,3 | 403,00 |
| s.rel in % | 2,56 | 8,41 |
| Lackauftragsmenge [mg] | 8,70 | N.B.* |
| Zerfallszeit, 37°C TABLTK [sek] | 28 | 22 |
| Zerfallszeit, 37°C FITAB [sek] | 74 (+164%) | 175 (+696%) |
| Friabilität TABLTK [%] | 0,45 | 14,71 |
| Friabilität FITAB [%] | 0,00 | 0,00 |

| | | |
|---|---|---|
| *Die Lackauftragsmenge konnte nicht ermittelt werden, da die Tablettenkerne während der Lackierung auf Grund der mechanischen Belastung und deren nur geringen Stabilität bröckelten | | |

### 1.4.1. Vergleich Aussehen der Filmtabletten Verarbeitungsbeispiel Nr. 4 mit Vergleichsbeispiel Nr. 4

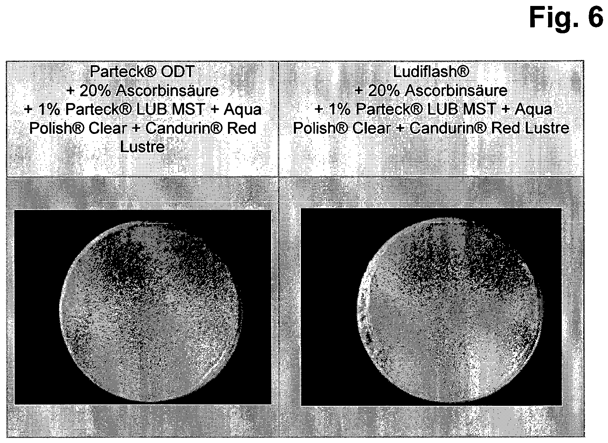

**Fig. 6****:** Vergleich Aussehen FITAB Parteck® ODT Verum und Ludiflash® Verum lackiert mit Aqua Polish® Clear

Während die aus Parteck® ODT hergestellte Filmtablette einen relativ gleichmäßigen Rand aufweist, zeigt die aus Ludiflash hergestellte Filmtablette erhebliche Abbrüche am Rand.

### 1.4.2. Fazit des Vergleichs Verarbeitungsbeispiel Nr. 2 mit Vergleichsbeispiel Nr. 2

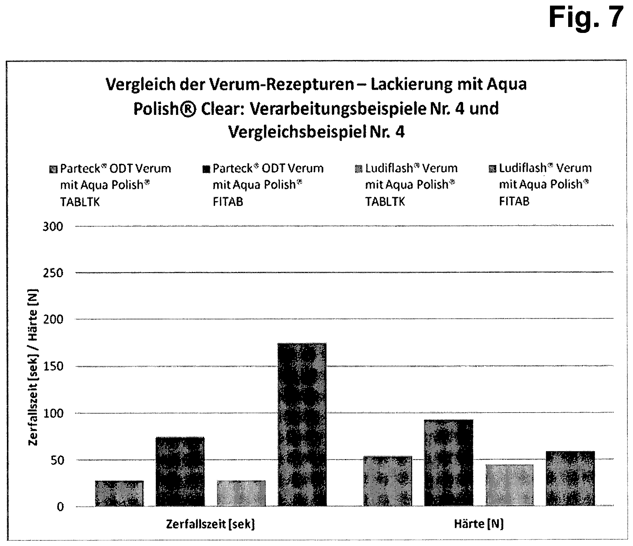

**Fig. 7****:** Darstellung der Auswertung des Vergleichs Verarbeitungsbeispiel Nr. 4 und Vergleichsbeispiel Nr. 4

Die Parteck® ODT Placbeo Tablettenkerne haben bei einer Härte von 54N eine Zerfallszeit von 28 Sekunden, ähnliche Eigenschaften zeigen auch die Ludiflash® Placebo Tablettenkerne mit einer Zerfallszeit von 22 Sekunden bei einer Tablettenhärte von 45N.

Nach der Lackierung mit Opadry™ 200 White sind allerdings signifikant Unterschiede festzustellen. So steigt die Härte der Parteck® ODT Placbeo Filmtabletten auf 93N (+72%) und die Zerfallszeit beträgt trotzdem nur 74 Sekunden (+164%). Im Gegenzug steigt die Zerfallszeit der Ludiflash® Placebo Filmtabletten auf 175 Sekunden (+696%) bei einer Härte von 59N (+31%).

### 1.5. Auswirkung der Lackierung auf die Zerfallszeit und Härte der Verarbeitungs- und Vergleichsbeispiele

### 1.5.1. Auswirkungen der Lackierung auf die Zerfallszeit und Härte der Placebo Verarbeitungs- und Vergleichsbeispiele

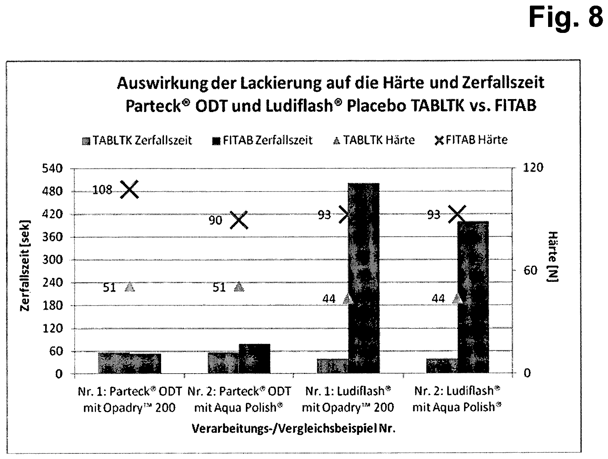

**Fig. 8****:** Auswirkungen der Placebo-Formulierungen im Vergleich

Während sich mit dem Aufbringen des Lackfilms auf die Parteck® ODT-Tabletten die Zerfallszeiten nur unwesentlich ändern, steigt bei Ludiflash®-Tabletten die Zerfallszeit auf mehr als 300 Sekunden, so dass es sich nicht mehr um eine schnell zerfallende Tablette handelt.

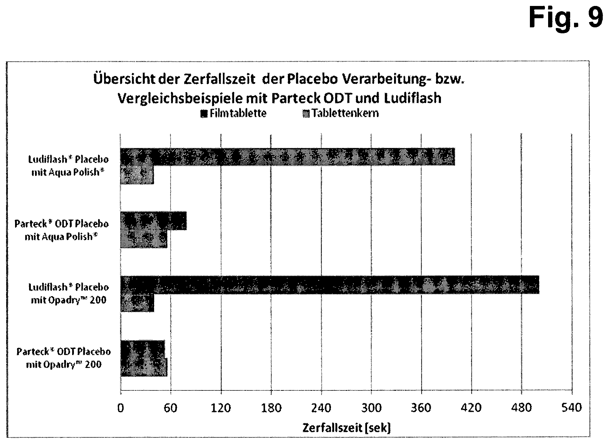

**Fig. 9****:** Betrachtung der Zerfallszeit der Placebo-Formulierungen im Vergleich
Hier gilt das gleiche wie bei Fig. 9

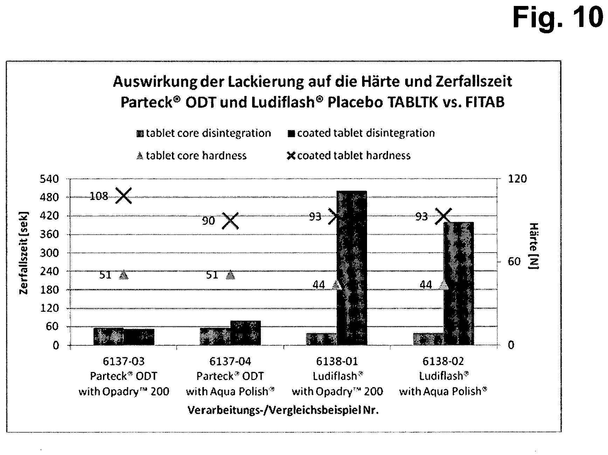

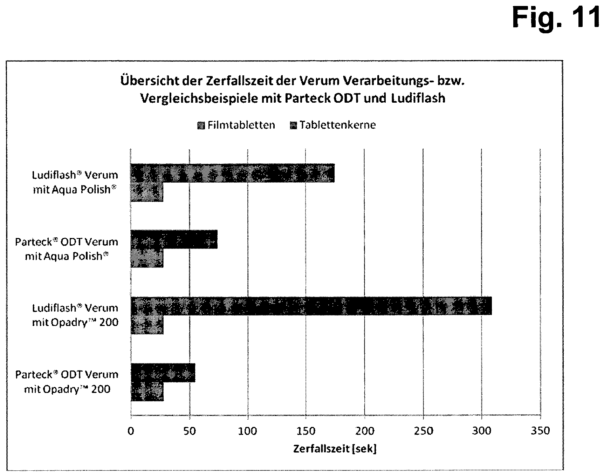

**Fig. 10****:** Auswirkung der Verum-Formulierungen im Vergleich

**Fig**. **11****:** Betrachtung der Zerfallszeit der Verum-Formulierungen im Vergleich

### 2. Vergleich der Lackierungsversuche mit Tablettenkernen, hergestellt aus den Fertigmischungen Parteck®ODT (Fa. Merck) und Ludiflash® (Fa. BASF) mit Lackierungszusammensetzungen auf Wasser-Ethanol- Basis

### 2.1. Vergleich der Placebo-Rezepturen - Lackierung mit Readi Lycoat D klar 590.03 G: Verarbeitungsbeispiele Nr. 10 und Vergleichsbeispiel Nr. 5

### Verarbeitungsbeispiel Nr. 10:

Beschichtung einer Placebo-Tablette (Parteck ODT) mit einem 5%igen Anteil an Fertiglack ReadiLycoat (Hersteller Biogrund GmbH, D-65510 Hünstetten, Deutschland)

### Vergleichsbeispiel Nr. 5:

Beschichtung einer Placebo-Tablette (Mitbewerberprodukt, Ludiflash, BASF) mit einem 5%igen Anteil an Fertiglack ReadiLycoat (Hersteller Biogrund GmbH, D-65510 Hünstetten, Deutschland)

**Tabelle 6: Vergleich des Verarbeitungsbeispiele Nr. 10 mit Vergleichsbeispiel Nr. 5**

| Parameter | Parteck® ODT + 1% Parteck® LUB MST + 5% Readi Lycoat | Ludiflash® + 1% Parteck® LUB MST + 5% Readi Lycoat |
|---|---|---|
| TABLTK Härte n. 1 Tag [N] | 51 | 44 |
| s.rel in % | 7,89 | 7,31 |
| FITAB Härte n. 1 Tag [N] | 91 (+78%) | 72 (+64%) |
| s.rel in % | 6,76 | 9,94 |
| Tablettengewicht [mg] | 399,95 | 407,83 |
| s.rel in % | 0,65 | 0,55 |
| Filmtablettengewicht [mg] | 399,70 | 408,10 |
| s.rel in % | 0,50 | 0,48 |
| Lackauftragsmenge [mg] | -0,25 | +0,27 |
| Zerfallszeit, 37°C TABLTK [sek] | 56 | 40 |
| Zerfallszeit, 37°C FITAB [sek] | 49 (-13%) | 61 (+53%) |
| Friabilität TABLTK [%] | 0,37 | 0,36 |
| Friabilität FITAB [%] | 0,21 | 0,23 |

### Vergleich der äußeren Erscheinung der Filmtabletten des Verarbeitungsbeispiels Nr. 10 und des Vergleichsbeispiels Nr. 5

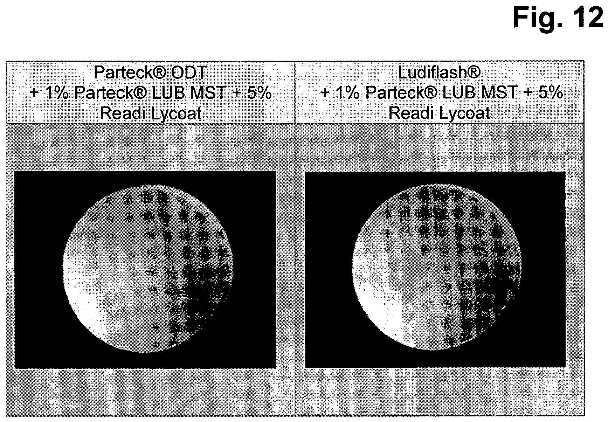

**Fig. 12****:** Vergleich Aussehen FITAB Parteck® ODT Placebo und Ludiflash® Placebo lackiert mit Readi Lycoat

### 2.1.1. Fazit des Vergleichs Verarbeitungsbeispiel Nr. 10 mit Vergleichsbeispiel Nr. 5

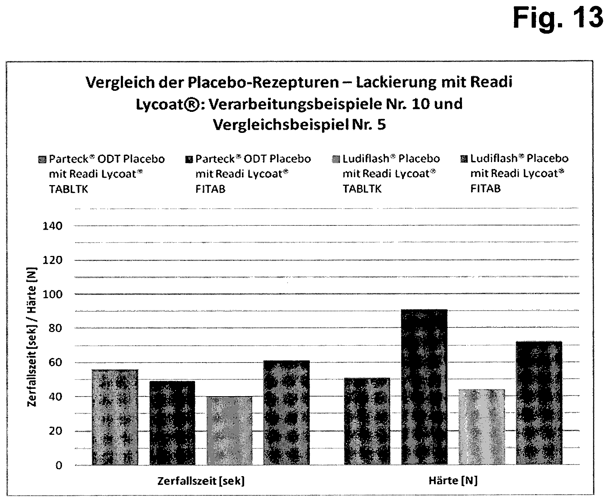

**Fig. 13****:** Darstellung der Auswertung des Vergleichs Verarbeitungsbeispiel Nr. 10 und Vergleichsbeispiel Nr. 5

### 2.2. Vergleich von wirkstoffhaltigen Tabletten - Lackierung mit Readi Lycoat D klar 590.03 G: Verarbeitungsbeispiele Nr. 11 und Vergleichsbeispiel Nr. 6

### Verarbeitungsbeispiel Nr. 11

Beschichtung einer Verum-Tablette (Parteck ODT) mit einem 5%igen Anteil an Fertiglack ReadiLycoat (Hersteller Biogrund GmbH, D-65510 Hünstetten, Deutschland)

### Vergleichsbeispiel Nr. 6:

Beschichtung einer Verum-Tablette (Mitbewerberprodukt, Ludiflash, BASF) mit einem 5%igen Anteil an Fertiglack ReadiLycoat (Hersteller Biogrund GmbH, D-65510 Hünstetten, Deutschland)

**Tabelle 7: Vergleich Verarbeitungsbeispiele Nr. 11 und Vergleichsbeispiel Nr. 6**

| Parameter | Parteck® ODT + 1% Parteck® LUB MST + 20% Ascorbinsäure + 5% Readi Lycoat | Ludiflash® + 1% Parteck® LUB MST + 20% Ascorbinsäure + 5% Readi Lycoat |
|---|---|---|
| TABLTK Härte n. 1 Tag [N] | 54 | 45 |
| s.rel in % | 7,14 | 10,24 |
| FITAB Härte n. 1 Tag [N] | 98 (+82%) | 46 (+2%) |
| s.rel in % | 7,06 | 14,36 |
| Tablettengewicht [mg] | 408,60 | 403,58 |
| s.rel in % | 0,58 | 0,60 |
| Filmtablettengewicht [mg] | 409,30 | 402,40 |
| s.rel in % | 0,84 | 0,48 |
| Lackauftragsmenge [mg] | +0,70 | N.B.* |
| Zerfallszeit, 37°C TABLTK [sek] | 28 | 22 |
| Zerfallszeit, 37°C FITAB [sek] | 27 (-4%) | 36 (+64%) |
| Friabilität TABLTK [%] | 0,45 | 14,71 |
| Friabilität FITAB [%] | 0,35 | 6,33 |

| | | |
|---|---|---|
| *Die Lackauftragsmenge konnte nicht ermittelt werden, da die Tablettenkerne während der Lackierung auf Grund der mechanischen Belastung und deren nur geringen Stabilität bröckelten. | | |

### 2.2.2. Vergleich Aussehen der Filmtabletten Verarbeitungsbeispiel Nr. 11 mit Vergleichsbeispiel Nr. 6

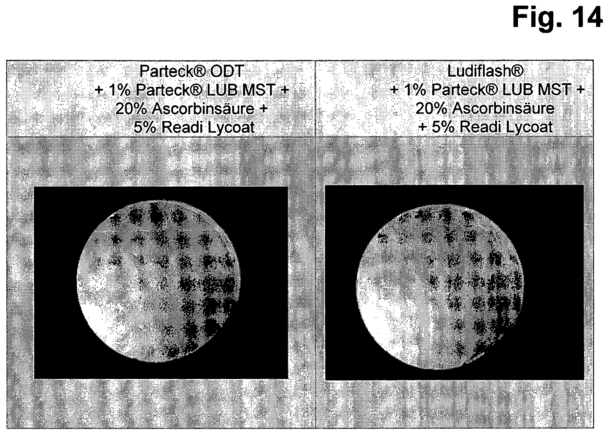

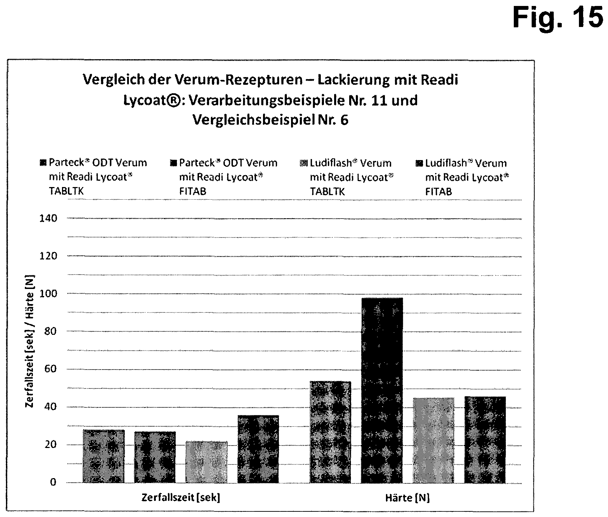

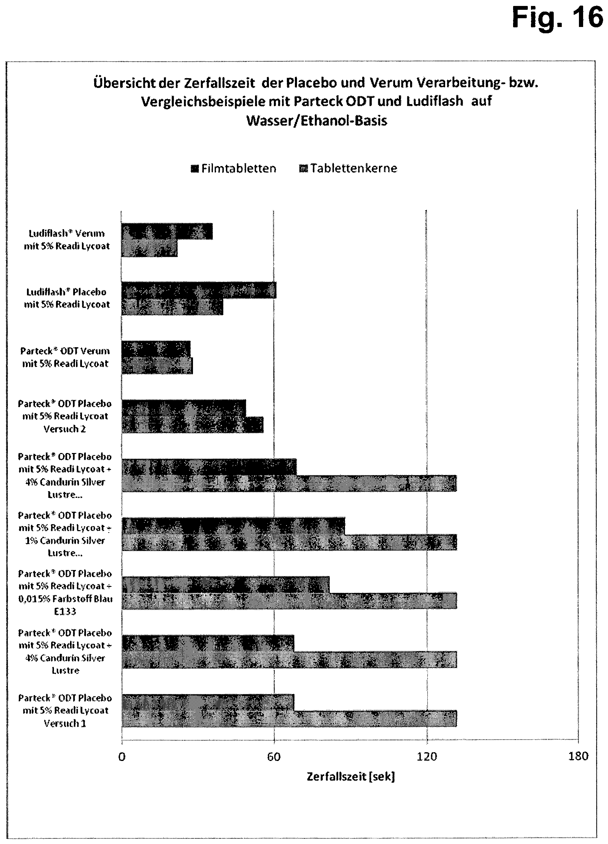

**Fig. 14****:** Vergleich Aussehen FITAB Parteck® ODT Verum und Ludiflash® Verumlackiert mit Readi Lycoat

Während die Ascorbinsäure-haltige Parteck® ODT- Filmtablette nur äußerst geringe Unregelmäßigkeiten am Rand aufweist, zeigt die entsprechende Ludiflash®-Tablette stark unregelmäßige Ränder und deutliche Unregelmäßigkeiten an der Oberfläche.

**Tabelle 8: Vergleich der physikalischen Produkteigenschaften zur Charakterisierung der verwendeten "ready to use" Systeme**

| **Parameter** | **Parteck® ODT Merck KGaA** | **Ludiflash® BASF SE** | **Pearlitol® Flash Roquette** |
|---|---|---|---|
| **Zusammensetzung** | **90 - 95% Mannit, 3-7% Croscarmellose-Natrium** | **84 - 92% Mannit, 4-6% Crospovidone, 3,5 - 6% Polyvinylacetate, 0,25 - 0,6% Povidone** | **80% Mannit, 20% Stärke** |
| **Herstellverfahren** | **Sprühagglomeration/ granulation & HomogenisierungsTechnologie** | **Co-Sprühtrocknung** | **Co-Sprühagglomeration bzw. Sprühtrocknung** |
| **Schüttdichte DIN EN ISO 60** | **0,59 g/ml** | 0,53 g/ml | **0,49 g/ml** |
| **Stampfdichte DIN EN ISO 787-11** | **0,75 g/ml** | 0,68 g/ml | **0,57 g/ml** |
| **Schüttwinkel DIN ISO 4324** | **35°** | 35° | **30°** |
| **Oberfläche BET-Methode** | **3,4654 m²/g** | **0,3049 m²/g** | **0,2798 m²/g** |
| **Porenvolumen BET-Methode** | **0,024368 cm³/g** | **0,000783 cm³/g** | **0,001275 cm³/g** |

| **Parameter** | **PROSOLV® ODT JRS Pharma** | **F-Melt® TypeC Fuji Chemical** | **F-Melt® Type M Fuji Chemical** |
|---|---|---|---|
| **Zusammensetzung** | **30 - 40% Mannit, 30 - 40% Fructose, 15 - 30% Mikrokristalline Cellulose, 4 - 6% Crospovidone, 1,5 - 2,5% Hochdisperses Silicumdioxid** | **65% Mannit, Xylit, Mikrokristalline Cellulose, Crospovidone, Magnesium-Aluminometa-silicate (Neusilin®)** | **65% Mannit, Xylit, Mikrokristalline Cellulose, Calciumhydrogenphosphat-Dihydrat (Fujicalin®)** |
| **Herstellverfahren** | **Co-processing Technology** | **Co-Sprühtrocknung** | **Co-Sprühtrocknung** |
| **Schüttdichte DIN EN ISO 60** | 0,63 g/ml | 0,57 g/ml | 0,61 g/ml |
| **Stampfdichte DIN EN ISO 787-11** | 0,80 g/ml | 0,68 g/ml | 0,71 g/ml |
| **Schüttwinkel DIN ISO 4324** | 34° | 31° | **28°** |
| **Oberfläche BET-Methode** | **1,7920 m²/g** | **0,6351 m²/g** | 3,2952 m²/g |
| **Porenvolumen BET**-**Methode** | **0,010844 cm³/g** | **0,003622 cm³/g** | 0,017061 cm³/g |

| **Parameter** | **Parteck® ODT Merck KGaA** | **Ludiflash® BASF SE** | **Pearlitol® Flash Roquette** |
|---|---|---|---|
| **REM-Aufnahme, 50x (300µm)** | 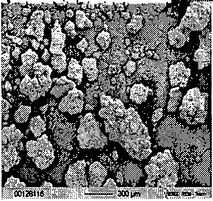 | 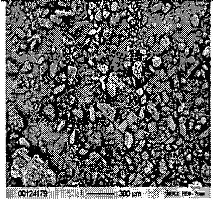 | 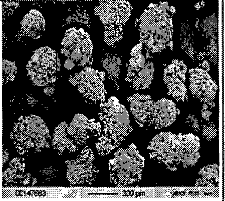 |
| **Wassergehalt nach Karl-Fischer-Titration** | **0,12%** | **0,92%** | Bestimmung nicht möglich! → Stärke-lod Reaktion |
| | (Spec. Value ≤ 2,0 %) | (Spec. Value max. 4,0 %) | |
| **Trocknungsverlust bei 105 °C nach 3 Stunden** | **0,15%** | **0,70 - 0,78%** | **1,34%** (1,0% laut CoA) |
| **Korngrößen-verteilung mit der Turmsiebung (Trockenmessung)** | **zwischen 32** - **300 µm: 78%** | zwischen 32 - 300 µm: 88% | zwischen 32 - 300 µm: 84% |
| **Korngrößen-verteilung mit der Laserbeugung (Trockenmessung)** | **Dᵥ₅₀, 1bar: 109µm** | **Dᵥ₅₀,,** 1bar: 76µm | **Dᵥ₅₀, 1bar: 23µm** |
| | **Dᵥ₅₀, 2bar: 79µm** | **Dᵥ₅₀, 2bar: 27µm** | **Dᵥ₅₀, 2bar: 11µm** |
| | **Dᵥ₅₀, 3bar: 73µm** | **Dᵥ₅₀, 3bar: 21µm** | **Dᵥ₅₀, 3bar: 10µm** |
| **Patentanmeldungen (Auflistung unvollständig!)** | WO2009152922 A1 | WO2008148734 A1 | WO20101001063 A1 |
| | | WO2007071581 A2 | |
| | | US20100184785 A1 | |

| **Parameter** | **PROSOLV® ODT JRS Pharma** | **F-Melt® Type C Fuji Chemical** | **F-Melt® Type M Fuji Chemical** |
|---|---|---|---|
| **REM-Aufnahme, 50x (300µm)** | 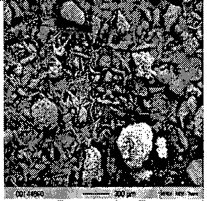 | 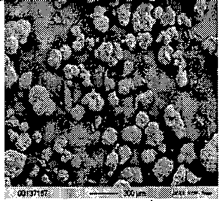 | 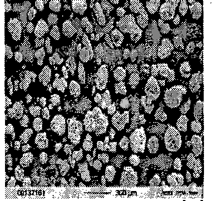 |
| **Wassergehalt nach Karl-Fischer-Titration** | **1,4** - **2,0%** laut CoA (Spec. Value max. 2,5%) | **1,01%** | **1,90%** |
| **Trocknungsverlust bei 105 °C nach 3 Stunden** | **0,91%** | **0,8-0,9%** | **1,63%** |
| | (2,0% laut CoA; Spec. Value max. 2,5%) | (1,0% laut CoA; Spec. Value 0,7-1,5%) | (1,7% laut CoA; Spec. Value 0,7-2,0%) |
| **Korngrößen-verteilung mit der Turmsiebung (Trockenmessung)** | zwischen | **zwischen** | **zwischen** |
| | 32 - 300 µm: | **32** - **300 µm:** | **32** - **300 µm:** |
| | 66% | **94%** | **96%** |
| **Korngrößen-verteilung mit der Laserbeugung (Trockenmessung)** | **Dᵥ₅₀,** 1bar: 88µm | **Dᵥ₅₀,** 1bar: 125µm | **Dᵥ₅₀,** 1bar: 121µm |
| | **Dᵥ₅₀, 2bar: 37µm** | **Dᵥ₅₀,** 2bar: 92µm | **Dᵥ₅₀,** 2bar: 100µm |
| | **Dᵥ₅₀, 3bar: 49µm** | **Dᵥ₅₀,**3bar: 90µm | **Dᵥ₅₀,3bar:** 88µm |
| **Patentanmeldungen (Auflistung unvoll-ständig!)** | **???** | JP No. 3841804 | JP No. 3841804 |
| | | **International Patents pending!** | **International Patents pending!** |
| | | EP 1 523 974 A1 | EP 1 523 974 A1 |
| | | EP 1 674 083 A1 | EP 1 674 083 A1 |

| | | | |
|---|---|---|---|
| Wesentliche bzw. signifikante Unterschiede zum Parteck® ODT sind **Fett** hervorgehoben! | | | |

**Tabelle 9: Galenische Eigenschaften der Tabletten bzw. Filmtabletten der eingesetzten ODT-Systeme**

| **Parameter** | **Parteck® ODT (Merck) + 1% Parteck® LUB MST + 2,15% Opadry™ 200 + 0,3% Colorona® Majectic Green** | **Ludiflash® (BASF) + 1% Parteck® LUB MST + 2,15% Opadry™ 200 + 0,3% Colorona® Majectic Green** | **Pearlitol® Flash (Roquette) + 1% Parteck® LUB MST + 2,15% Opadry™ 200 + 0,3% Colorona® Majectic Green** |
|---|---|---|---|
| **TABLTK Härte Inprozess [N] (Soll = 50N)** | 50 | 50 | 54 |
| **Relative Standardab-weichung [%]** | 7,93 | 7,90 | 6,67 |
| **TABLTK Härte n. 1 Tag [N]** | 51 | 44 | 46 |
| **Relative Standardab**w**eichung [%]** | 7,89 | 7,31 | 8,36 |
| **FITAB Härte n. 1 Tag [N]** | **108** (+112%) | **93** (+111%) | 106 (+130%) |
| **Relative Standardabweichung [%]** | 11,58 | 10,45 | 8,36 |

| **Parameter** | **PROSOLV® ODT (JRS Pharma) + 1% Parteck® LUB MST + 2,15% Opadry™ 200 + 0,3% Colorona® Majectic Green** | **F-Melt® Type C (Fuji Chemical) + 1% Parteck® LUB MST + 2,15% Opadry™ 200 + 0,3% Colorona® Majectic Green** | **F-Melt® Type M (Fuji Chemical) + 1% Parteck® LUB MST + 2,15% Opadry™ 200 + 0,3% Colorona® Majectic Green** |
|---|---|---|---|
| **TABLTK Härte Inprozess [N] (Soll = 50N)** | 51 | 50 | 55 |
| **Relative Standardabweichung [%]** | 7,65 | 7,42 | 7,59 |
| **TABLTK Härte n. 1 Tag [N]** | 44 | 45 | 53 |
| **Relative Standardabweichung [%]** | 11,05 | 8,90 | 7,45 |
| **FITAB Härte n. 1 Tag [N]** | 112 (+155%) | 113 (+151%) | **139** (+162%) |
| **Relative Standardabweichung [%]** | 11,05 | 8,90 | 7,45 |

| **Parameter** | **Parteck® ODT (Merck) + 1% Parteck® LUB MST + 2,15% Opadry™ 200 + 0,3% Colorona® Majectic Green** | **Ludiflash® (BASF) + 1% Parteck® LUB MST + 2,15% Opadry™ 200 + 0,3% Colorona® Majectic Green** | **Pearlitol® Flash (Roquette) + 1% Parteck® LUB MST + 2,15% Opadry™ 200 + 0,3% Colorona® Majectic Green** |
|---|---|---|---|
| **TABLTK-Masse [mg] (Soll = 400mg)** | 400,0 | 407,8 | 401,0 |
| **Relative Standardabweichung [%]** | 0,65 | 0,55 | 0,48 |
| **Zerfallszeit, 37°C TABLTK [sek]** | **56** | **40** | **54** |
| **Zerfallszeit, 37°C FITAB [sek]** | **53 (-5%)** | **501 (+1153%)** | **80 (+48%)** |
| **Friabilität TABLTK [%]** | **0,37** | 0,36 | 0,33 |
| **Friabilität FITAB [%]** | 0,00 | 0,00 | 0,00 |
| **Aussehen FITAB** | glatt, geringe Beschädigungen am Rand, mintgrün/weiß gesprenkelt, glänzend | glatt, geringe Beschädigungen am Rand, mintgrün/weiß gesprenkelt, leicht glänzend | glatt, vereinzelt Abrieb an den Kanten, mintgrün/weiß gesprenkelt, leicht glänzend |

| **Parameter** | **PROSOLV® ODT (JRS Pharma) + 1% Parteck® LUB MST + 2,15% Opadry™ 200 + 0,3% Colorona® Majectic Green** | **F-Melt® Type C (Fuji Chemical) + 1% Parteck® LUB MST + 2,15% Opadry™ 200 + 0,3% Colorona® Majectic Green** | **F-Melt® Type M (Fuji Chemical) + 1% Parteck® LUB MST + 2,15% Opadry™ 200 + 0,3% Colorona® Majectic Green** |
|---|---|---|---|
| **TABLTK-Masse [mg] (Soll = 400mg)** | 402,4 | 407,5 | 404,0 |
| **Relative Standardabweichung [%]** | 0,64 | 0,74 | 0,52 |
| **Zerfallszeit, 37°C TABLTK [sek]** | **115** | **17** | 66 |
| **Zerfallszeit, 37°C FITAB [sek]** | **201 (+75%)** | **79 (+365%)** | **138 (+109%)** |
| **Friabilität TABLTK [%]** | **0,67** | 0,30 | **1,34** |
| **Friabilität FITAB [%]** | 0,00 | 0,00 | 0,00 |
| **Aussehen FITAB** | etwas rauh, mintgrün/weiß gesprenkelt, leicht glänzend | glatt, mintgrün/weiß gesprenkelt, leicht glänzend | glatt, mintgrün/weiß gesprenkelt, leicht glänzend |

| | | | |
|---|---|---|---|
| TABLTK = Tablettenkern (Tablettenkern vor Lackierung), FITAB = Filmtablette (Tablettenkern nach Lackierung → Lackierte Filmtablette) Wesentliche bzw. signifikante Unterschiede zum Parteck® ODT sind Fett hervorgehoben! | | | |

## Patentansprüche

1. Pharmazeutische Formulierung in Form einer Tablette mit Überzug, **dadurch gekennzeichnet, dass** sie aus
a) einem Tablettenkern, erhältlich aus einer homogenisierten, direkt verpressbaren Co-Mischung von sprühgranuliertem Mannit und quervernetzter Croscarmellose-Natrium als Tablettensprengmittel und mindestens einem pharmazeutischen Wirkstoff oder Nahrungsergänzungsmittel und Additiven,
und
b) einem Überzug, der in Form einer wässrigen oder einer Wasser und Alkohol haltigen Lösung aufgebracht wird,
besteht und dass es sich um eine in Gegenwart von Feuchtigkeit schnell zerfallende Tablette handelt.

2. Pharmazeutische Formulierung gemäß Anspruch 1, **dadurch gekennzeichnet, dass** die zur Herstellung des Tablettenkerns verwendete Co-Mischung 90 bis 95 Gew.-% Mannit und 3 bis 7 Gew.-% Croscarmellose-Natrium als Tablettensprengmittel, und gegebenenfalls bis zu 1 Gew.-% Magnesiumstearat enthält.

3. Pharmazeutische Formulierung gemäß Anspruch 1 und 2, **dadurch gekennzeichnet, dass** die zur Herstellung des Tablettenkerns verwendete Co-Mischung einen Fließwinkel im Bereich von 33 bis 38°, Partikelgrößen im Bereich von 70 bis 120 µm (D₅₀; Laser), eine Schüttdichte im Bereich von 0,55 bis 0,65 g/ml und eine Stampfdichte im Bereich von 0,70 bis 0,80 g/ml aufweist.

4. Pharmazeutische Formulierung gemäß Anspruch 1 und 2, **dadurch gekennzeichnet, dass** die zur Herstellung des Tablettenkerns verwendete Co-Mischung eine BET-Oberfläche im Bereich von 2,4 bis 3,5 m²/g aufweist.

5. Pharmazeutische Formulierung gemäß einem oder mehreren der Ansprüche 1 bis 4, **dadurch gekennzeichnet, dass** der Tablettenkern einen pharmazeutischen Wirkstoff oder Nahrungsergänzungsmittel in einer Menge von 0,1 bis zu 50 Gew-% bezogen das Gewicht des Tablettenkerns enthält.

6. Pharmazeutische Formulierung gemäß einem oder mehreren der Ansprüche 1 bis 5, **dadurch gekennzeichnet, dass** der Tablettenkern Gleit- bzw. Schmiermittel in Form von Magnesiumstearat, Natriumstearylfumarate, Stearinsäure, Polyethylenglycol (PEG 6000) in einer Menge von bis zu 0,1 bis 5 Gew.-% bezogen das Gewicht des Tablettenkerns enthält.

7. Pharmazeutische Formulierung gemäß einem oder mehreren der Ansprüche 1 bis 5, **dadurch gekennzeichnet, dass** der Überzug in Form einer Wasser- oder Wasser-Ethanol- haltigen Lösung aufgebracht wird.

8. Pharmazeutische Formulierung gemäß einem oder mehreren der Ansprüche 1 bis 5, **dadurch gekennzeichnet, dass** der Überzug lösliche Filmbildner aus der Gruppe Polyvinylpyrrolidon, Vinylpyrrolidon / Vinylacetat Copolymer, Polyvinylacetat, Hydroxypropylmethylcellulose, Methacrylestercopolymer oder deren Mischungen enthält.

9. Pharmazeutische Formulierung gemäß einem oder mehreren der Ansprüche 1 bis 5, **dadurch gekennzeichnet, dass** der Überzug aus einer Lösung hergestellt wird, die neben einem oder mehreren Filmbildnern einen oder mehrere Zucker aus der Gruppe Glucose, Dextrose, Fructose, Laktose, Maltose, Xylose, Sucrose, Maissirup, Sorbit, Hexitol, Maltit, Xylit und Mannit, gegebenenfalls mindestens einen Polyalkohol ausgewählt aus der Gruppe Glycerin, Polyethylenglykol und Propylenglykol, sowie gegebenenfalls mindestens eine essbare, für Lebensmittel geeignete Säure aus der Gruppe Zitronensäure, Äpfelsäure, Weinsäure, Fumarsäure, Phosphorsäure, Oxalsäure und Ascorbinsäure, und Aromaöle und/oder Geschmacksstoffe enthalten, die sich bereits während des Lösens des äußeren Tablettenüberzugs im Mund angenehmen auswirken.

10. Pharmazeutische Formulierung gemäß einem oder mehreren der Ansprüche 1 bis 9, **dadurch gekennzeichnet, dass** der Tablettenkern einen Wirkstoff ausgewählt aus der Gruppe Atypical antipsychotics, Antipsychotics, Antidepressants, Anti-histamines, Acetylcholinesterase inhibitors, Analgesics, Anti-pyretics, Anticonvulsant, Anticholinergic, Antiemetics, Benzodiazepines, Corticosteroids, DDC inhibitors [carbidopa], Dopamine receptor antagonists, Monoamine oxidase inhibitors (MAOIs), Nonbenzodiazepine Hypnotics, Opioid analgesic [Tramadol], Proton pump inhibitors, Triptans/Serotonin agonists, NSAIDsund SSRIs enthält.

11. Pharmazeutische Formulierung gemäß einem oder mehreren der Ansprüche 1 bis 10, **dadurch gekennzeichnet, dass** der Tablettenkern einen niedrigen Abrieb von weniger als 0,50 % bezogen auf das Gewicht aufweist.

12. Verfahren zur Herstellung einer pharmazeutischen Formulierung gemäß einem oder mehreren der Ansprüche 1 bis 10, **dadurch gekennzeichnet, dass** die hergestellten, in Gegenwart von Wasser schnell zerfallenden Tablettenkerne in einer Lackiertrommel unter Durchmischung auf eine erhöhte Temperatur erwärmt werden und der Überzug durch Aufsprühen der niedrigviskosen Überzugslösung und Trocknen bei erhöhter Temperatur auf den Tablettenkernen erzeugt wird.

13. Verfahren gemäß Anspruch 12, **dadurch gekennzeichnet, dass** die Tablettenkerne vor dem Aufsprühen der Überzugslösung auf eine Temperatur im Bereich von 35 bei 60 °C, vorzugsweise im Bereich von 40 bis 55°C, erwärmt werden.

14. Verfahren gemäß der Ansprüche 12 und 13, **dadurch gekennzeichnet, dass** die Tabletten nach dem Aufsprühen der Überzugslösung 10 bis 20 Minuten nachgetrocknet werden.

## Claims

1. Pharmaceutical formulation in the form of a coated tablet, **characterised in that** it consists of
a) a tablet core, obtainable from a homogenised, directly compressible co-mixture of spray-granulated mannitol and crosslinked croscarmellose-sodium as tablet disintegrant and at least one pharmaceutical active compound or food supplement and additives,
and
b) a coating which is applied in the form of an aqueous or water- and alcohol-containing solution,
and **in that** it is a tablet which disintegrates rapidly in the presence of moisture.

2. Pharmaceutical formulation according to Claim 1, **characterised in that** the co-mixture used for the production of the tablet core comprises 90 to 95% by weight of mannitol and 3 to 7% by weight of croscarmellose-sodium as tablet disintegrant, and optionally up to 1% by weight of magnesium stearate.

3. Pharmaceutical formulation according to Claims 1 and 2, **characterised in that** the co-mixture used for the production of the tablet core has a flow angle in the range from 33 to 38°, particle sizes in the range from 70 to 120 µm (D₅₀; laser), a bulk density in the range from 0.55 to 0.65 g/ml and a tapped density in the range from 0.70 to 0.80 g/ml.

4. Pharmaceutical formulation according to Claims 1 and 2, **characterised in that** the co-mixture used for the production of the tablet core has a BET surface area in the range from 2.4 to 3.5 m²/g.

5. Pharmaceutical formulation according to one or more of Claims 1 to 4, **characterised in that** the tablet core comprises a pharmaceutical active compound or food supplement in an amount of 0.1 to 50% by weight, based the weight of the tablet core.

6. Pharmaceutical formulation according to one or more of Claims 1 to 5, **characterised in that** the tablet core comprises glidants or lubricants in the form of magnesium stearate, sodium stearyl fumarate, stearic acid or polyethylene glycol (PEG 6000) in an amount of up to 0.1 to 5% by weight, based the weight of the tablet core.

7. Pharmaceutical formulation according to one or more of Claims 1 to 5, **characterised in that** the coating is applied in the form of a water- or water/ethanol-containing solution.

8. Pharmaceutical formulation according to one or more of Claims 1 to 5, **characterised in that** the coating comprises soluble film formers from the group polyvinylpyrrolidone, vinylpyrrolidone-vinyl acetate copolymer, polyvinyl acetate, hydroxypropylmethylcellulose, methacrylate copolymer or mixtures thereof.

9. Pharmaceutical formulation according to one or more of Claims 1 to 5, **characterised in that** the coating is produced from a solution which, besides one or more film formers, comprises one or more sugars from the group glucose, dextrose, fructose, lactose, maltose, xylose, sucrose, corn syrup, sorbitol, hexitol, maltitol, xylitol and mannitol, optionally at least one polyalcohol selected from the group glycerol, polyethylene glycol and propylene glycol, and optionally at least one edible acid which is suitable for foods, from the group citric acid, malic acid, tartaric acid, fumaric acid, phosphoric acid, oxalic acid and ascorbic acid, and aroma oils and/or flavours, which have a pleasant effect in the mouth even during dissolution of the outer tablet coating.

10. Pharmaceutical formulation according to one or more of Claims 1 to 9, **characterised in that** the tablet core comprises an active compound selected from the group atypical antipsychotics, antipsychotics, antidepressants, antihistamines, acetylcholinesterase inhibitors, analgesics, antipyretics, anticonvulsants, anticholinergics, antiemetics, benzodiazepines, corticosteroids, DDC inhibitors [carbidopa], dopamine receptor antagonists, monoamine oxidase inhibitors (MAOIs), nonbenzodiazepine hypnotics, opioid analgesics [tramadol], proton pump inhibitors, triptans/serotonin agonists, NSAIDs and SSRIs.

11. Pharmaceutical formulation according to one or more of Claims 1 to 10, **characterised in that** the tablet core has low abrasion of less than 0.50%, based on the weight.

12. Process for the preparation of a pharmaceutical formulation according to one or more of Claims 1 to 10, **characterised in that** the tablet cores produced, which disintegrate rapidly in the presence of water, are warmed to an elevated temperature in a coating drum with mixing, and the coating is produced on the tablet cores by spraying on the low-viscosity coating solution and drying at elevated temperature.

13. Process according to Claim 12, **characterised in that** the tablet cores are warmed to a temperature in the range from 35 to 60°C, preferably in the range from 40 to 55°C, before the coating solution is sprayed on.

14. Process according to Claims 12 and 13, **characterised in that** the tablets are dried for 10 to 20 minutes after the coating solution has been sprayed on.

## Revendications

1. Formulation pharmaceutique sous la forme d'un comprimé enrobé, **caractérisée en ce qu'**elle est constituée par :
a) un noyau de comprimé, qui peut être obtenu à partir d'un co-mélange homogénéisé directement compressible de mannitol granulé par atomisation et de croscarmellose sodique réticulé en tant qu'agent de désintégration de comprimé et d'au moins un composé actif pharmaceutique ou un complément alimentaire et des additifs,
et
b) un enrobage qui est appliqué sous la forme d'une solution aqueuse ou contenant de l'eau et de l'alcool,
et **en ce qu'**il s'agit d'un comprimé qui se désintègre rapidement en présence d'humidité.

2. Formulation pharmaceutique selon la revendication 1, **caractérisée en ce que** le co-mélange qui est utilisé pour la fabrication du noyau de comprimé comprend de 90 % à 95 % en poids de mannitol et de 3 % à 7 % en poids de croscarmellose sodique en tant qu'agent de désintégration de comprimé, et en option, jusqu'à 1 % en poids de stéarate de magnésium.

3. Formulation pharmaceutique selon les revendications 1 et 2, **caractérisée en ce que** le co-mélange qui est utilisé pour la fabrication du noyau de comprimé présente un angle d'écoulement à l'intérieur de la plage qui va de 33° à 38°, des tailles de particule à l'intérieur de la plage qui va de 70 µm à 120 µm (D₅₀; laser), une masse volumique apparente à l'intérieur de la plage qui va de 0,55 g/ml à 0,65 g/ml et une densité après tassement à l'intérieur de la plage qui va de 0,70 à 0,80 g/ml.

4. Formulation pharmaceutique selon les revendications 1 et 2, **caractérisée en ce que** le co-mélange qui est utilisé pour la fabrication du noyau de comprimé présente une aire de surface BET à l'intérieur de la plage qui va de 2,4 m²/g à 3,5 m²/g.

5. Formulation pharmaceutique selon une ou plusieurs des revendications 1 à 4, **caractérisée en ce que** le noyau de comprimé comprend un composé actif pharmaceutique ou un complément alimentaire selon une quantité de 0,1 % à 50 % en poids, sur la base du poids du noyau de comprimé.

6. Formulation pharmaceutique selon une ou plusieurs des revendications 1 à 5, **caractérisée en ce que** le noyau de comprimé comprend des agents de glissement ou des lubrifiants sous la forme de stéarate de magnésium, de fumarate de stéaryle sodique, d'acide stéarique ou de polyéthylène glycol (PEG 6000) selon une quantité qui va jusqu'à 0,1 % à 5 % en poids, sur la base du poids du noyau de comprimé.

7. Formulation pharmaceutique selon une ou plusieurs des revendications 1 à 5, **caractérisée en ce que** l'enrobage est appliqué sous la forme d'une solution contenant de l'eau ou contenant de l'eau/de l'éthanol.

8. Formulation pharmaceutique selon une ou plusieurs des revendications 1 à 5, **caractérisée en ce que** l'enrobage comprend des agents filmogènes solubles pris parmi le groupe constitué par le polyvinylpyrrolidone, le copolymère vinylpyrrolidone-acétate de vinyle, l'acétate de polyvinyle, l'hydroxypropylméthylcellulose, un copolymère de méthacrylate ou des mélanges de ceux-ci.

9. Formulation pharmaceutique selon une ou plusieurs des revendications 1 à 5, **caractérisée en ce que** l'enrobage est produit à partir d'une solution qui, en plus d'un ou de plusieurs agent(s) filmogène(s), comprend un ou plusieurs sucre(s) pris parmi le groupe constitué par le glucose, le dextrose, le fructose, le lactose, le maltose, le xylose, le sucrose, le sirop de glucose, le sorbitol, l'hexitol, le maltitol, le xylitol et le mannitol, en option au moins un polyalcool sélectionné parmi le groupe constitué par le glycérol, le polyéthylène glycol et le propylène glycol, et en option, au moins un acide comestible qui est approprié pour les aliments, pris parmi le groupe constitué par l'acide citrique, l'acide malique, l'acide tartarique, l'acide fumarique, l'acide phosphorique, l'acide oxalique et l'acide ascorbique, et des huiles aromatiques et/ou des arômes, lesquel(le)s présentent un effet plaisant en bouche même pendant la dissolution de l'enrobage externe du comprimé.

10. Formulation pharmaceutique selon une ou plusieurs des revendications 1 à 9, **caractérisée en ce que** le noyau de comprimé comprend un composé actif sélectionné parmi le groupe constitué par les antipsychotiques atypiques, les antipsychotiques, les antidépresseurs, les antihistaminiques, les inhibiteurs d'acétylcholinestérase, les analgésiques, les antipyrétiques, les anticonvulsifs, les anticholinergiques, les antiémétiques, les benzodiazépines, les corticostéroïdes, les inhibiteurs de DDC [carbidopa], les antagonistes de récepteur de dopamine, les inhibiteurs de la monoamine oxydase (MAOI), les hypnotiques non benzodiazépine, les analgésiques opioïdes [tramadol], les inhibiteurs de pompe à protons, les triptanes/agonistes sérotoniniques, les NSAID et les SSRI.

11. Formulation pharmaceutique selon une ou plusieurs des revendications 1 à 10, **caractérisée en ce que** le noyau de comprimé présente une abrasion faible inférieure à 0,50%, sur la base du poids.

12. Procédé pour la préparation d'une formulation pharmaceutique selon une ou plusieurs des revendications 1 à 10, **caractérisé en ce que** les noyaux de comprimé produits, qui se désintègrent rapidement en présence d'eau, sont chauffés jusqu'à une température élevée dans un tambour d'enrobage tout en effectuant un mélange, et l'enrobage est produit sur les noyaux de comprimé par atomisation dessus de la solution d'enrobage à faible viscosité et par séchage à température élevée.

13. Procédé selon la revendication 12, **caractérisé en ce que** les noyaux de comprimé sont chauffés jusqu'à une température à l'intérieur de la plage qui va de 35°C à 60°C, de préférence à l'intérieur de la plage qui va de 40°C à 55°C, avant que la solution d'enrobage ne soit atomisée dessus.

14. Procédé selon les revendications 12 et 13, **caractérisé en ce que** les comprimés sont séchés pendant 10 à 20 minutes après que la solution d'enrobage a été atomisée dessus.
